# EUROPEAN PATENT APPLICATION

(11) **EP 1 162 460 A1**
(43) Date of publication of application: **12.12.2001**
(21) Application number: 00112479.1
(22) Date of filing: 09.06.2000
(51) Int. Cl.: G01N 33/68, C07K 16/40, C12N 9/10, C12Q 1/48

(54) **Mammalian Suv39h2 proteins and isolated DNA molecules encoding them**

(71) Applicant: Boehringer Ingelheim International GmbH, 55218 Ingelheim am Rhein (DE)
(72) Inventor: Jenuwein, Thomas, Dr., 1030 Wien (AT); O'Carroll, Donal, Dr., Wicklow (IE); Rea, Stephen, Dr., Galway (IE)
(74) Representative: Laudien, Dieter, Dr.

(57) **Abstract**

Murine and human Suv39h2 polypeptide and DNA molecules encoding them. *Suv39h2* is a novel member of the *Suv3-9* gene family. Suv39h2 is a novel component of meiotic higher order chromatin. It has histone methyltransferase activity and is required, in combination with Suv39hl, for male gametogenesis. Suv39h2 can be used in screening methods to identify modulators of its methyltransferase activity, which are useful for male contraception.

## Description

fold (Ball *et al*., 1997), the structure of the 130 amino acid SET domain (Jenuwein *et al*., 1998) is currently undefined. However, it has recently been shown that the SET domain of Suv39h1 harbours an intrinsic HMTase activity, which is specific for lysine 9 of histone H3 (Rea *et al*., 2000). These data suggest that *Suv39h* homologues exert their function through the organisation chromatin structure via histone H3 methylation.

The corresponding mouse (*Suv39h1*) and human (*SUV39H1*) *Su(var)3-9* homologues have been identified and it has been demonstrated that *SUV39H1* represents a functional mammalian homologue of *Su(var)3-9* in transgenic flies (Aagaard *et al*., 1999). Immunolocalisation of endogenous Suv39h1 or SUV39H1 proteins in mammalian cells indicated enriched distribution at heterochromatic foci during interphase and transient accumulation at centromeric positions during mitosis (Aagaard *et al*., 2000). In addition, Suv39h1 or SUV39H1 associate with M31 (HP1β), one mammalian homologue of *Drosophila* HP1, indicating the existence of a mammalian SU(VAR) protein complex(es) (Aagaard *et al*., 1999). Moreover, deregulated SUV39H1 can induce ectopic heterochromatin and redistribute endogenous M31 (HP1β) (Melcher *et al*., 2000). These data defined Suv39h1 or SUV39H1 as novel heterochromatic HMTase proteins that are involved in the structural organisation of mammalian higher-order chromatin in somatic cells.

It was the object of the invention to further identify other mammalian *Su(var)3-9* homologues and to investigate their function through gene expression, protein immunolocalisation analysis and gene disruption techniques in the mouse.

To solve the problem underlying the present invention, the following approaches were taken.

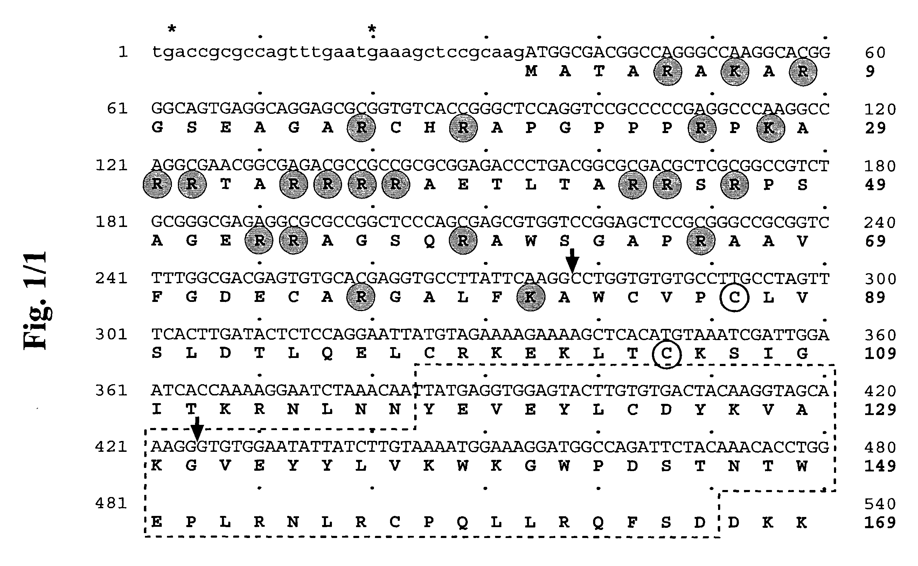

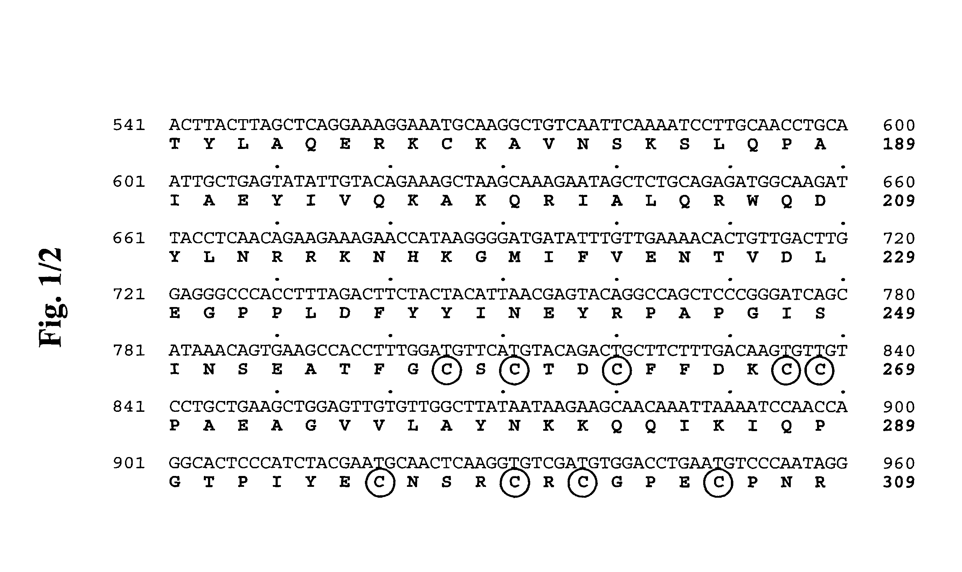

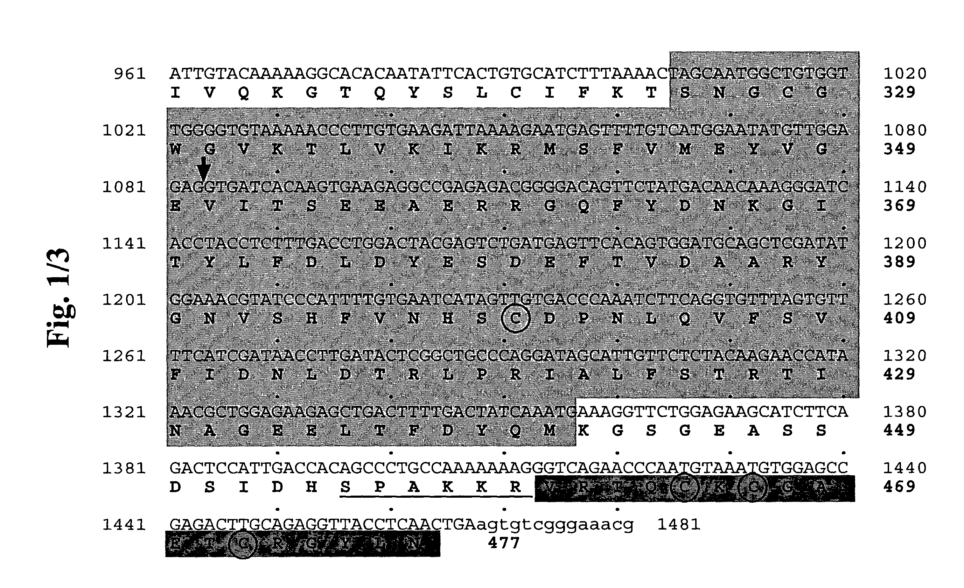

To identify additional mammalian *Su(var)3-9* homologues, sequence similarity searches (Bassett *et al*., 1995; Altschul *et al*., 1997) with the murine *Suv39h1* or human *SUV39H1* cDNAs (Aagaard *et al*., 1999) revealed the presence additional Su(var)3-9 homologue. In analogy to *Suv39h1*, this novel gene was designated *Suv39h2* (for *Su(var)3-9* homologue 2). The nucleotide sequence (∼ 1.5 kb) and conceptional reading frame (477 amino acids) of the composite coding *Suv39h2* cDNA is shown in Figure 1.

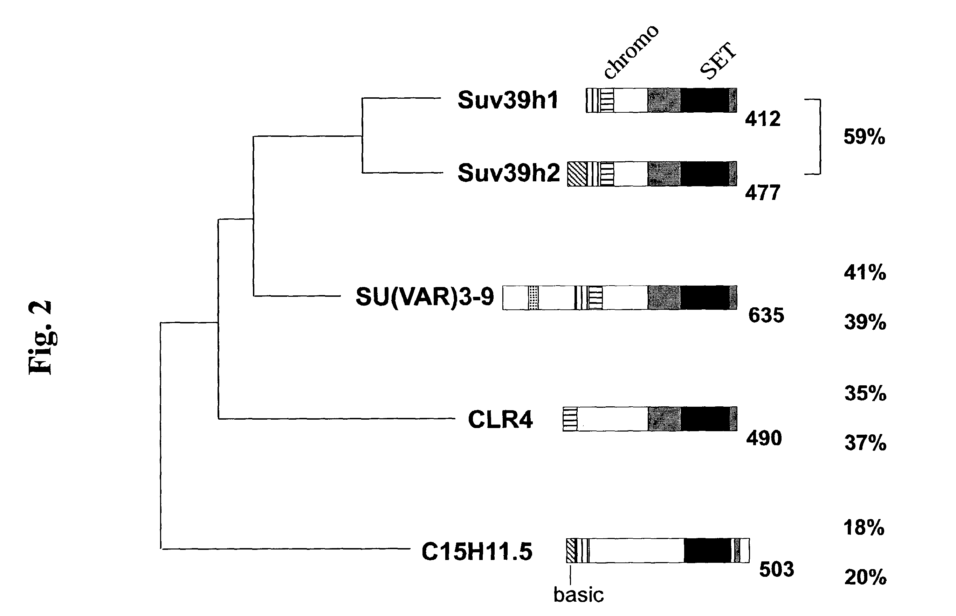

Cross-species comparison of Suv39h2 with Suv39h1 or other representative members of the SU(VAR)3-9 protein family, like *Drosophila* SU(VAR)3-9 (Tschiersch *et al*., 1994), *S.pombe* CLR4 (Ivanova *et al*., 1998) and a putative open reading frame (ORF) in *C.elegans* (C15H11.5; accession number Z81035) indicate very similar sequence identities and phylogenetic relationships (Figure 2).

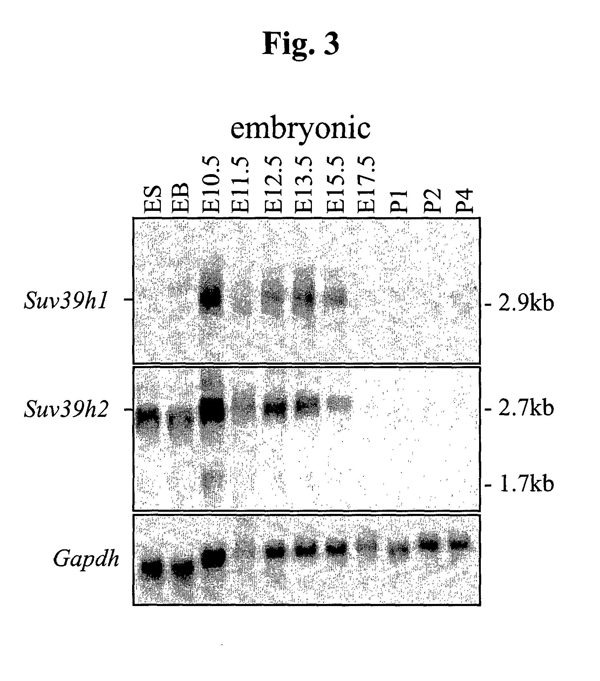

To determine the size *of Suv39h2* mRNAs, RNA blots containing total RNA from embryonic stem cells (ES-cells) and mouse embryos from various stages (day E10.5 - day E17.5) of embryogenesis and postnatal (P1 - P4) development were hybridised with a 980 bp cDNA probe comprising *Suv39h2* coding sequences (amino acids 143-477) and a near full length *Suv39h1* cDNA probe. Within this region, the *Suv39h2* cDNA is approximately 60% identical to the *Suv39h1* nucleotide sequence and does not cross-hybridise with *Suv39h1* transcripts (see Figure 3 and data not shown). This *Suv39h2*-specific cDNA probe detected a prominent mRNA of approximately 2.7 kb in most RNA preparations of the analysed stages (Figure 3A, middle panel). The size of the great majority of *Suv39h2* transcripts agrees with a 2.7 kb mRNA also found in several mouse and human cell lines (data not shown), whereas only at day E10.5, smaller-sized (1.7 kb) transcripts were detected.

Expression analysis of both *Suv39h1* and *Suv39h2* revealed potential overlapping functions during embryogenesis. Northern blot and whole-mount RNA in situ analysis were used to determine the embryonic expression profiles of *Suv39h1* and *Suv39h2.* Both genes are ubiquitously expressed during embryogenesis.

Expression analysis revealed potential distinct functions for both *Suv39h1* and *Suv39h2* in the adult mouse. In contrast to embryonic expression profiles, abundance of *Suv39h2* and *Suv39h1* transcripts greatly differs in adult tissues.

Whereas *Suv39h1* displays broad expression in a panel of RNA preparations comprising 14 adult tissues, expression of *Suv39h2* remains largely restricted to testes, with mRNAs being present as 2.7 kb and 1.7 kb transcripts.

To characterise Suv39h2 expression at a biochemical level, a polyclonal rabbit antiserum was generated that was raised against a recombinant glutathione *S*-transferase (GST) fusion protein comprising amino acids 157-477 of murine Suv39h2. The anti-Suv39h2 antibodies recognise an endogenous protein of approximately 53 kDa in both PMEFs and testis. The size of the endogenous Suv39h2 protein is in good agreement with the gene product predicted from the coding sequence of the *Suv39h2* cDNA (see Figure 1).

In order to elucidate a potential function for Suv39h2 in male gametogenesis, the subnuclear localisation endogenous Suv39h2 protein in nuclei of testis swab preparations was analysed (see Materials and Methods) by indirect immunofluorescence with the anti-Suv39h2 antibodies. Suv39h2 is a component of meiotic heterochromatin and the XY body during mid pachytene.

To demonstrate the specific accumulation of Suv39h2 with the sex chromosomes, double immunofluorescence analyses for Suv39h2 and SCP3, for Suv39h2 and Xmr, and for Suv39h2 and Hit was performed. These analyses revealed specific association of Suv39h2 with sex chromosomes from mid-late pachytene to diplotene.

It has been shown in parallel experiments that the SET domain of Suv39h1 harbours an intrinsic HMTase activity. It was therefore analysed whether other SU(VAR)3-9 family members, in particular Suv39h2, or other SET domain proteins exhibit HMTase activity. GST-fusion products of the extended SET domains of murine Suv39h2, *S.pombe* CLR4 (Ivanova *et al*., 1998), human EZH2 (Laible *et al*., 1997) and human HRX (Tkachuk *et al*., 1992) were generated that would correspond to GST-SUV39H1(82-412) and HMTase activity assayed. Interestingly, GST-Suv39h2(157-477) and GST-CLR4(127-490) also displayed HMTase activity. These data identify Suv39h2 as a novel component of meiotic chromatin, the XY body and as a meiotic histone H3 MTase.

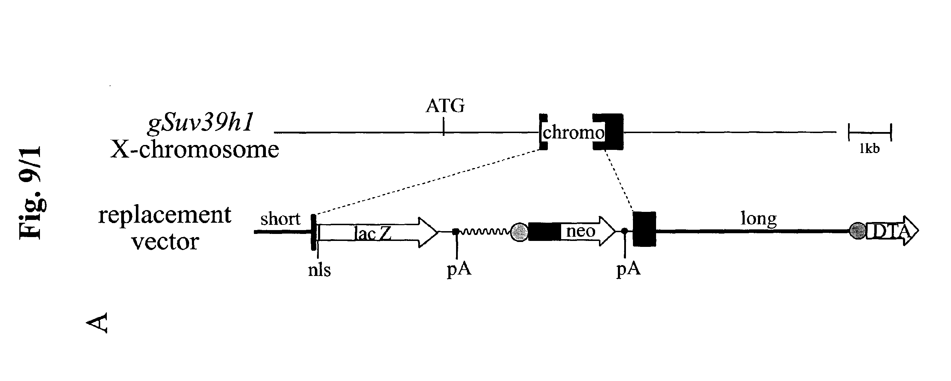

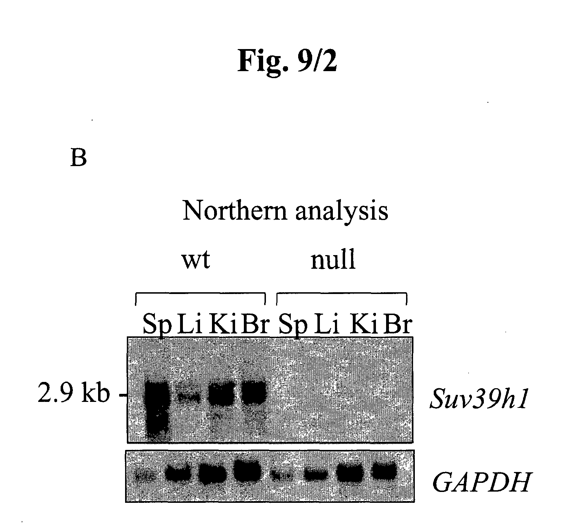

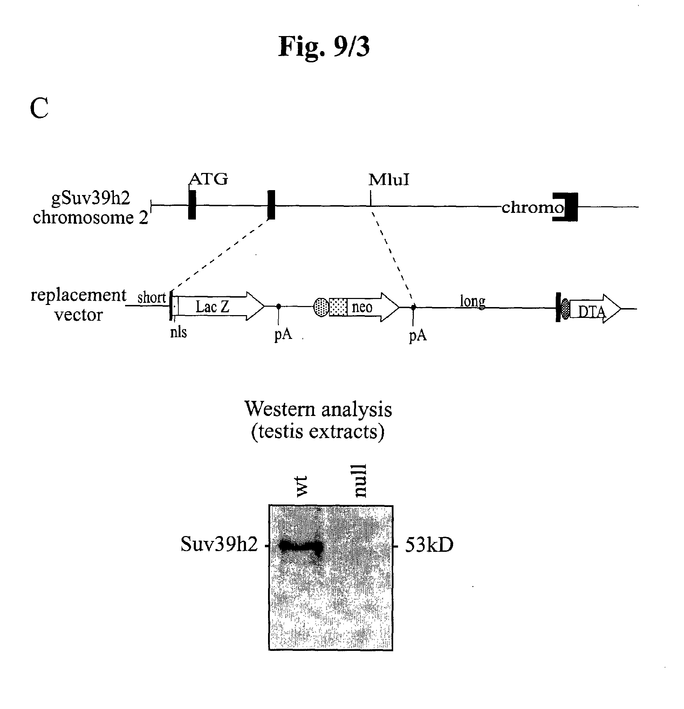

To investigate the *in vivo* significance of *Suv39h* function, in particular *Suv39h2* function in male gametogenesis, mouse strains deficient for both *Suv39h1* and *Suv39h2* were generated according to standard techniques. The targeting strategies are shown in Figure 9, as well as demonstrating the production of null alleles for both *Suv39h1* and *Suv39h2*. Mutation of either gene results in viable and fertile mice as a consequence of functional redundancy between both loci. Therefore, *Suv39h1* and *Suv39h2* deficient strains were intercrossed to produce *Suv39h* double deficient mice.

Double mutant mice are born in sub-Mendelian ratios, approximately 20% of the expected double mutants are observed (data not shown) and are infertile. These data identify the *Suv39h* genes as essential regulators of higher order mammalian chromatin in chromosomal dynamics during male meiosis/gametogenesis. In addition, they identify *Suv39h2* as a novel tool in the treatment of male infertility and as a target for reversible male contraception.

In a first aspect, the present invention relates to the murine Suv39h2 polypeptide with the amino acid sequence as set forth in SEQ ID NO:2 or with the amino acid sequence encoded by a polynucleotide which hybridises under stringent conditions to a polynucleotide having a nucleotide sequence as set forth in SEQ ID NO:1.

By "stringent hybridisation conditions" as used herein is meant overnight incubation at 42°C in a solution comprising: 50% formamide, 5x SSC (1X SSC = 150 mM NaCl, 15mM trisodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C, or equivalent conditions.

In a further aspect, the present invention relates to an isolated DNA molecule comprising a polynucleotide with the nucleotide sequence as set forth in SEQ ID NO:1 encoding murine Suv39h2 polypeptide or an isolated DNA molecule encoding murine Suv39h2, comprising a polynucleotide which hybridises under stringent conditions to a polynucleotide having a nucleotide sequence as set forth in SEQ ID NO:1.

In a preferred embodiment, the invention relates to the human SUV39H2 polypeptide encoded by a polynucleotide containing the sequence of the human EST accession number AQ173625 (SEQ ID NO:3) and/or AQ494637 (SEQ ID NO:4) and/or AQ691972 (SEQ ID NO:5) and/or AQ554070 (SEQ ID NO:6), or by a polynucleotide which hybridises under stringent conditions to the said polynucleotides.

In a further aspect, the present invention relates to an isolated DNA molecule encoding the human SUV39H2 protein, comprising a polynucleotide containing the sequence of the human EST accession number AQ173625 (SEQ ID NO:3) and/or AQ494637 (SEQ ID NO:4) and/or AQ691972 (SEQ ID NO:5) and/or AQ554070 (SEQ ID NO:6), or an isolated DNA molecule.

The sequence information in the ESTs AQ173625, AQ494637, AQ691972 and AQ554070 partially define human *SUV39H2.* The corresponding human *SUV39H2* cDNA can be readily isolated using sequence information in the AQ173625, AQ494637, AQ691972 and AQ554070. The ESTs or part of the ESTs can be used as a probe to screen a suitable phage cDNA library, such as a testis library. Otherwise the sequence information in the above mentioned ESTs could be used to design a PCR (RT-PCR or RACE amplification) based strategy to isolate SUV39H2.

In the following, if not otherwise stated, the term " Suv39h2" refers to both the murine and the human SUV39H2.

Homologues of the subject Suv39h2 proteins also include versions of the polypeptide which are resistant to post-translation modification or which alter an enzymatic activity of the protein. The Suv39h2 polypeptide can comprise a full length protein, such as represented in SEQ ID NO:2, or it can comprise a fragment or variant thereof.

Beside DNA molecules, the present invention relates to nucleic acid molecules in the form of RNA, such as mRNA. The DNA molecules include cDNA and genomic DNA obtained by cloning or produced synthetically. The DNA may be double-stranded or single-stranded. Single-stranded DNA or RNA may be the coding strand, also known as the sense (or plus) strand, or it may be the non-coding strand, also referred to as the antisense (or minus) strand.

By "isolated" nucleic acid molecule(s) is intended a nucleic acid molecule, DNA or RNA, which has been removed from its native environment. Recombinant DNA molecules contained in a vector are considered isolated for the purposes of the present invention. Further examples of isolated DNA molecules include recombinant DNA molecules maintained in heterologous host cells, and those DNA molecules purified (partially or substantially) from a solution whether produced by recombinant DNA or synthetic chemistry techniques. Isolated RNA molecules include *in vivo* or *in vitro* RNA transcripts of the DNA molecules of the present invention. However, it is intended that "isolated" as used herein does not include the *Suv39h2* cDNA present in a cDNA library or in a preparation of purified or isolated genomic DNA containing the *Suv39h2* gene or a portion thereof in admixture with one or more other cDNA molecules or DNA fragments.

The nucleic acid molecules of the present invention further include genetic constructs comprising one or more *Suv39h2* DNA sequences operably linked to regulatory DNA sequences (which may be heterologous regulatory sequences), such as promoters or enhancers as described below, wherein upon expression of these DNA sequences in host cells, preferably in bacterial, fungal (including yeast), plant or animal (including insect or mammalian) cells, one or more Suv39h2 polypeptides are produced. In such constructs, the regulatory sequences may be operably linked to a *Suv39h2* polynucleotide encoding mature Suv39h2 polypeptide or any of its variants, precursors, fragments or derivatives described herein, which may include one or more polynucleotides having a nucleic acid sequence that is complementary to substantially all or a portion of a nucleic acid molecule having a nucleic acid sequence as shown in SEQ ID NO: 1, 3, 5 and 6. As used herein, the terms "a portion" or "a fragment" of a nucleic acid molecule or a polypeptide means a segment of a polynucleotide or a polypeptide comprising at least 15, and more preferably at least 20, contiguous nucleotides or amino acids of a reference polynucleotide or polypeptide (for example, the polynucleotide and polypeptide shown in SEQ ID NOs: 1, 2 or 3 and 4, respectively, unless otherwise specifically defined below.)

Besides the DNA molecules having a nucleotide sequence corresponding to that depicted SEQ ID NO: 1, or containing a sequence of SEQ ID NO: 3 and/or 4 and/or 5 and/or 6; the invention also relates to DNA molecules which comprise a sequence substantially different from those described above but which, due to the degeneracy of the genetic code, still encode the Suv39h2 mouse or human polypeptides. Since the genetic code is well known in the art, it is routine for one of ordinary skill in the art to produce the degenerate variants described above without undue experimentation.

In addition, the invention relates to Suv39h2 polypeptides which have deviations from the sequence shown in SEQ ID NO:2 or from a polypeptide encoded by a polynucleotide containing a sequence of SEQ ID NO: 3 and/or 4 and/or 5 and/or 6, caused by the conservative exchange of amino acids, if they are Suv39h2 derivatives or fragments or peptides with the properties which are desirable for their use in therapy or in screening assays. The invention also relates to isolated DNA molecules encoding such derivatitives or fragments with a polynucleotide sequence varying in their sequence from SEQ ID NO:1, or isolated DNA molecules varying in their sequence from a polynucleotide containing a sequence of SEQ ID NO: 3 and/or 4 and/or 5 and/or 6.

Nucleic acid molecules of the present invention which encode a Suv39h2 polypeptide or a derivative or fragment thereof may include, but are not limited to, those encoding the amino acid sequence of the polypeptide by itself, together with additional, non-coding sequences, including for example introns and non-coding 5' and 3' sequences, such as the transcribed, untranslated regions (UTRs) or other 5' flanking sequences that may play a role in transcription *(e.g.,* via providing ribosome- or transcription factor-binding sites), mRNA processing *(e.g.* splicing and polyadenylation signals) and stability of mRNA; the coding sequence for the *Suv39h2* polypeptide operably linked to a regulatory DNA sequence, particularly a heterologous regulatory DNA sequence such as a promoter or enhancer; and the coding sequence for the *Suv39h2* polypeptide linked to one or more coding sequences which code for amino acids that provide additional functionalities. Thus, the sequence encoding the polypeptide may be fused to a marker sequence, such as a sequence encoding a peptide which facilitates purification of the fused polypeptide. In certain embodiments of this aspect of the invention, the marker amino acid sequence may be a hexa-histidine peptide, such as the tag provided in a pQE vector (Qiagen, Inc.), among others, many of which are commercially available. As described for instance in Gentz et al., 1989. The "HA" tag is another peptide useful for purification which corresponds to an epitope derived from the influenza hemagglutinin protein, which has been described by Wilson *et al*., 1984. Yet another useful marker peptide for facilitation of purification of *Suv39h2* is glutathione S-transferase (GST) encoded by the pGEX fusion vector (see, e.g., Winnacker, From Genes to Clones, New York: VCH Publishers, pp. 451-481 (1987)). As discussed below, other such fusion proteins include the *Suv39h2* fused to immunoglobulin Fc at the N- or C-terminus.

A still further aspect of the present invention relates to antibodies and antibody preparations specifically reactive with an epitope of the Suv39h2 polypeptide.

Polyclonal antibodies are conventionally obtained by immunising animals, particularly rabbits, by injecting the antigen or fragments thereof and subsequently purifying the immunoglobulin.

Monoclonal anti-Suv39h2 antibodies may be obtained by standard procedures following the principle described by Köhler and Milstein, 1975, by immunising animals, particularly mice, then immortalising antibody-producing cells from the immunised animals, e.g. by fusion with myeloma cells, and screening the supernatant of the hybridomas obtained by immunological standard assays for monoclonal anti- Suv39h2 antibodies. For therapeutic or diagnostic use in humans, these animal antibodies may optionally be chimerised in the conventional way (Neuberger et al., 1984, Boulianne et al., 1984, or humanised (Riechmann et al., 1988, Graziano et al., 1995).

Suv39h2 specific antibodies can be used to understand higher order chromatin mediated chromosome dynamics and for screening human conditions for Suv39h2 mediated pathologies.

The invention also features transgenic non-human animals, e.g., mice, rats, rabbits, chickens, frogs or pigs, having a transgene, e.g., animals which include (and preferably express) a heterologous form of an *Suv39h2* gene described herein, or which mis-express an endogenous *Suv39h2* gene, e.g. an animal in which expression of one or more of the *Suv39h* genes are disrupted. Such animals can serve as a model for studying cellular and tissue disorders comprising mutated or mis-expressed *Suv*39h2 alleles or for drug screening.

Another aspect of the present invention provides a method of determining if a subject, e.g., a human patient, is at risk for a disorder characterised by unwanted cell proliferation or aberrant control of differentiation. The method includes detecting, in a tissue of the subject, the presence or absence of a genetic lesion characterised by a mutation or a mis-expression of the *Suv39h2* gene. In preferred embodiments, detecting the genetic lesion includes asserting the existence of at least one of: a deletion of one or more nucleotides from a *Suv39h* gene; an addition of one or more nucleotides to the gene, a substitution of one or more nucleotides of the gene, a cross chromosomal rearrangement of the gene; an alteration in the level of a messenger RNA transcript of a gene; the presence of a non-wild type splicing pattern of a messenger RNA transcript of the gene; or a non-wild type level of the protein.

The expression and immunolocalisation studies conducted in the present invention identify Suv39h2 as a novel component of meiotic higher order chromatin and the XY body. It has also been shown that the Suv39h homologues Suv39h1 and suv39h2 possess histone methyltransferase (HMTse) activity and that *Suv39h* function, supplied by Suv39h2, presumably in cooperation with Suv39h1, is an absolute requirement for male gametogenesis. The experiments of the present invention identify the Suv39h homologues Suv39h2 and, optionally, Suv39h1, as targets for novel strategies for reversible inhibition of male gametogenesis.

Due to their identification as K9 specific histone H3 MTases and as a requirement for male gametogenesis, Suv39h homologues are also useful in a method for identifying compounds that have the ability of modulating mammalian male gametogenesis. This method is characterised in that one or more Suv39h homologues required for male gametogenesis are incubated, in the presence of the substrate(s) for the HMTase activity and in the presence of a methyl donor, with test compounds and that the modulating effect of the test compounds on the HMTase activity of the Suv39h homologue(s) is determined.

In a preferred embodiment, Suv39h2 is employed in a primary screen, most preferably in its recombinant form. In a next step, the compound identified in the primary screen to be a modulator, e.g. an inhibitor, of Suv39h2, is assayed in a secondary screen for its ability to modulating, e.g. inhibit, a further Suv39h homologue that is required for male gametogenesis, in particular Suv39h1. This secondary screen is identical to the one described above for Suv39h2.

Suv39h homologues can be produced recombinantly according to standard methods by expression in suitable hosts, e.g. bacteria, yeast, insect or eucaryotic cells and purified, e.g. on glutathione-agarose columns if it has been tagged with GST.

For testing compounds for their effect on Suv39h activity, the assay comprises, as its essential features, incubating a histone H3 protein or histone H3 N-terminal fragment including K9, a methyl donor, e.g. methionine or S-adenosyl-L-methionine, with a preparation containing Suv39h and determining the HMTase of activity in the presence or absence of a test substance.

Useful substrates may be those equivalent to or mimicking the naturally occurring substrates, e.g. biochemically purified histone H3, recombinantly produced histone H3, or an histone H3 peptide that contains the K9 methylation site.

Preferably, the histone H3 fragment ARTKQTARKSTGGKAPRKQL (SEQ ID NO:7) is employed.

The methyl donor preferably carries a detectable label, e.g. a radioactive or a chromogenic label, which can be quantified upon transfer to the substrate.

Preferably, the methyl donor is the natural methyl donor S-adenosyl-L-Methionine. Alternatively to using a labelled methyl donor, the substrate, upon methylation by the enzyme, serves as an epitope which can be recognised by a specific antibody and hence used for quantification by standard immunoassay techniques, e.g. ELISAs. Antibodies useful in this type of assay can be obtained by using the methylated substrate, preferably a small peptide, e.g. the K9 methylated peptide ARTKQTARKSTGGKAPRKQL (SEQ ID NO:7) as an antigen and obtaining polyclonal or monoclonal antibodies according to standard techniques.

The assay, which may also be conducted on a high-throughput scale, is based on the catalytic transfer, mediated by a Suv39h39 homologue, of a methyl group from a substrate to a histone H3 peptide. To achieve this, the substrate, e.g. histone H3 or a variant or fragment thereof, is immobilised on a carrier, usually a microtiter plate, and incubated with recombinant Suv39h and a methyl donor. The methyl donor preferably carries a chromogenic or radioactive label. Upon transfer of the methyl group to the substrate by Suv39h, in the case of a chromogenic reagent, the methyl donor changes colour which can quantified. In the case of using a radioactive methyl donor, the methyl group is transferred to the substrate and can be quantified.

Alternatively, the methylation of the substrate can be quantified by ELISA using an antibody specific for the methylated substrate. If a test substance is an inhibitor of the MTase activity, there will be, depending on the detection system and depending on whether the test substance has an inhibiting or an activating effect, a decrease or an increase in the detectable signal.

In the high-throughput format, compounds with a modulationg effect Suv39h activity can be identified by screening test substances from compound libraries according to known assay principles, e.g. in an automated system on microtiter plates.

The compounds identified in the above methods have the ability to interfere with male gametogenesis by modulating the histone H3 methyl transferase activity of Suv39h homologues required for male gametogenesis.

These compounds, which act as modulators of Suv39h, can be used as reversible modulators of male gametogenesis, i.e. inhibitors can be used for male contraception.

The efficacy of compounds identified as Suv39h modulators can be tested for *in vivo* efficacy in mammals. The compound can be administered to adult male mice and fertility assayed.

As *Suv39h2* is required to maintain a stable karyotype, it can be considered as possessing tumour suppressor gene activity. If *Suv39h2* mutation proves to be a factor underlying cellular transformation events, the re-introduction of a wild type *Suv39h* gene by gene therapy may result in increased genomic stability delaying or inhibiting cancer progression.

In addition, the *Suv39h* loss of function studies demonstrate that *Suv39h* has an essential function in male gametogenesis. Loss of *Suv39h* function may underlie a subset of male sterility cases in humans. Re-introduction of *Suv39h2* or *Suv39h1* genes into developing gametes through gene therapy has the potential to rectify these defects.

For gene therapy, the *Suv39h* DNA molecule may be administered, preferably contained on a plasmid in recombinant form, directly or as part of a recombinant virus or bacterium. In principle, any method of gene therapy may be used for applying *Suv39h* recombinant DNA, both *in vivo* and *ex vivo.*

Examples of *in vivo* administration are the direct injection of "naked" DNA, either by intramuscular route or using a gene guns. Examples of recombinant organisms are vaccinia virus or adenovirus. Moreover, synthetic carriers for nucleic acids such as cationic lipids, microspheres, micropellets or liposomes may be used for *in vivo* administration of nucleic acid molecules coding for the Suv39h2 polypeptide.

Brief description of figures:
Figure 1: The coding part and conceptional reading frame of the *Suv39h2* cDNA.
Figure 2: Conserved domains of *S.pombe*, *C.elegans*, *Drosophila* and murine SU(VAR)3-9 related proteins.
Figure 3: Expression of *Suv39h1* and *Suv39h2* during mouse development.
Figure 4: Testis-specific expression of *Suv39h2.*
Figure 5: Detection and size of the endogenous Suv39h2 protein.
Figure 6: Dynamic heterochromatin association of Suv39h2 during most stages of spermatogenesis.
Figure 7: Suv39h2 accumulates with sex chromosomes present in the X-Y body.
Figure 8: The mammalian Su(var)3-9 harbours an intrinsic HMTase activity.
Figure 9: Targeting *Suv39h1* and *Suv39h2* in the mouse germline.
Figure 10: *Suv39h* function is required for male gametogenesis.

### Materials and Methods

### a) Molecular cloning of murine Suv39h2

A 210 bp EST DNA probe (encoding amino acids 219-289 of Suv39h2, see Figure 1) was PCR-amplified from murine B-cell specific (J558L and S194) cDNA libraries using the *Suv39h2*-EST primers 5' GGGGATGATATTTGTTGAAAACAC (SEQ ID NO:8) and 5' GGTTGGATTTTAATTTGTTGCTTC (SEQ ID NO:9). This *Suv39h2*-EST DNA probe was screened against a day E11.5 mouse embryonic λgt11 cDNA library (Clontech) and a λ129/Sv genomic library (Stratagene), resulting in the isolation of six cDNA and three genomic clones. The longest cDNA (1kb; λ4-*Suv39h2*) and genomic (14kb) isolates were sequenced by primer walking on an automated sequencer (Applied Biosystems). Sequence analysis indicated that the cDNA encoded amino acids 132-477, and that the genomic sequence comprised exons 1-3, as predicted by GENE-finder. Missing 5' sequences of the *Suv39h2* cDNA were extended by nested RACE amplification (Marathon cDNA amplification kit; Clontech) from the J558L and S194 cDNA libraries using the exon 3 specific primers 5' GCCCTCCAAGTCAACAGTG (SEQ ID NO: 10) and 5' GTGTTGAGGTAATCTTGCCATC (SEQ ID NO:11). The RACE amplifications identified exon 2 (amino acids 83-131). Exon 1, including the starting ATG, was deduced from an EST (accession number AA959164) which correctly spliced into exon 2 and whose sequence information was confirmed by comparison with genomic sequences.

### b) RNA isolation and analysis

RNA isolation and analysis was done as described previously (Laible *et al*., 1997; Aagaard *et al*., 1999). Membranes were sequentially hybridised under stringent Church conditions (Sambrook *et al*., 1989) with a 1.6 kb EcoRI cDNA fragment comprising nearly full-length *Suv39h1* or with a 980 bp cDNA PCR amplicon which codes for amino acid 143 to 477 of *Suv3h2*. To control for the quality of the RNA preparations, blots were rehybridised with a DNA probe that is specific for *Gadph* sequences (Dugaiczyk *et al*., 1983).

### c) In-situ analyses of Suv39h1 and Suv39h2 expression with RNA probes

To obtain *Suv39h1* and *Suv39h2*-specific riboprobes, PCR-converted *Sal*I/*Bam*HI DNA fragments were subcloned into the polylinker of pGEM-3Zf (Promega) which allows *in vitro* transcription by SP6 and T7 RNA polymerases. Similar to an internal 395 bp DNA fragment encoding amino acids 113-237 of Suv39h1 (Aagaard *et al*., 1999), a 325 bp internal DNA fragment encoding amino acids 186-290 of Suv39h2 was used. Within this region, *Suv39h1* and *Suv39h2* nucleotide sequences are only approximately 53% identical and do not cross-hybridise. *In-situ* RNA probes were internally labelled with DIG-UTP (Boehringer Mannheim) by transcription with SP6 (antisense probe of *Eco*RI linearised plasmid) or T7 RNA polymerase (sense probe of *Bam*HI linearised plasmid).

*In-situ* hybridizations of whole-mount embryos or of 5 µm sections of paraffin-embedded testis were performed at 65-70°C O/N, washed under high stringency and processed for detection after incubation with anti-DIG alkaline phosphatase-conjugated antibodies and BM purple as the chromogenic substrate (Boehringer Mannheim).

### d) Nuclear extracts and protein blot analysis

Isolation of nuclei from mouse testis was performed according to described protocols (Bunick *et al*., 1990; Motzkus *et al*., 1999). Approximately 30 µg of nuclear extracts from testis, the HeLa cell clones or from PMEFs were analysed on protein blots with anti-myc, anti-M31 (HP1β) (Wreggett *et al*., 1994), anti-Suv39h1 and anti-Suv39h2 antibodies as recently reported (Aagaard *et al*., 1999).

### e) Generation and purification of rabbit polyclonal anti-Suv39h2-specific antibodies

*Suv39h2* coding sequences comprising amino acids 157-477 were converted into a *Bam*HI*-Eco*RI DNA fragment by PCR amplification and combined in-frame with N-terminal glutathione-*S*-transferase (GST) in the bacterial expression vector pGEX-2T (Pharmacia). Purification of recombinant protein and immunisation of rabbits with the GST-Suv39h2 antigen was done as described (Aagaard *et al*., 1999). An IgG fraction was prepared from the crude serum of rabbit #2218, batch-preabsorbed against GST-Suv39h1 glutathione-Sepharose beads (Aagaard *et al*., 1999), and anti-Suv39h2 antibodies were affinity-purified over a glutathione-Sepharose (Pharmacia) column that had been loaded with GST-Suv39h2. Following elution with 100 mM glycine pH 2.5, antibodies were neutralised with 1/10 vol. of 2 M Hepes pH 7.9. These affinity-purified anti-Suv39h2 antibodies (concentration ∼ 0.5 mg/ml) were used at 1: 250 or 1: 500 dilutions for protein blot analysis or at 1:10 to 1: 20 dilutions for indirect immunofluorescence.

### f) Immunofluorescence analysis of testis suspension cells

Testes were surgically removed from 3-6 months old C57B16/129 mice and minced with scalpel blades in cold MEM medium (Gibco) containing protease inhibitors (Roche Biochemicals). Structurally preserved suspension cells were prepared by cross-linking fixation as described (Pandita *et al*., 1999). Testis suspension cells were mixed with equal volumes of PBS-buffered (pH 7.2) 3.7% formaldehyde, 0.1 M sucrose, placed on silanised glass slides and allowed to dry down until they were coated by a thin layer of sucrose.

For indirect immunofluorescence (IF) of Suv39h epitopes, sucrose-embedded cells were briefly washed with PBS, extracted for 30 min. with 0.2% Triton X-100, PBS and incubated O/N at 4°C with rabbit polyclonal anti-Suv39h1 (1:20; (Aagaard *et* *al*., 1999)) or rabbit polyclonal anti-Suv39h2 (1:20) antibodies that had been diluted in PTBG (PBS, 0.1% Tween 20, 0.2% BSA, 0.1% gelatin). Following three 3 min. washes in PTBG, samples were either incubated for 45 min. at 37°C with secondary, CY3-conjugated goat anti-rabbit antibodies (Vector Laboratories) or with secondary goat anti-rabbit biotinylated antibodies (1; 500; Dianova) that were visualized after a third incubation by Avidin-FITC (1: 1,000; Sigma). After three final washes in PBS, 0.1% Tween 20, preparations were mounted in Antifade solution (Vector Laboratories) containing 4',6'-diamidino-2-phenylindole (0.5mg/ml) (DAPI; Sigma). Specificity of the staining was confirmed by control IF analyses in the absence of primary antibodies. Staging of individual mouse spermatogenic cells was determined by the development of SCP3-positive axial cores and the specific distribution of heterochromatin (Scherthan *et al*., 1996).

For double-labelling experiments, samples were first incubated with anti-Suv39h2 (1:10) antibodies, followed by sandwich detection with anti-rabbit biotinylated antibodies and Avidin-CY3 or Avidin-FITC. After a brief fixation with 1% formaldehyde in PBS, SCP3 or H1t epitopes were then detected with rabbit polyclonal anti-SCP3 (1: 1,000; (Lammers *et al*., 1994)) or rabbit polyclonal anti-H1t (1:1,000; (Moens, 1995)) antibodies and visualised by secondary sheep anti-rabbit FITC-conjugated or sheep anti-rabbit CY3-conjugated (both Dianova) antibodies. Similarly, after triple-labelling for Suv39h2 with biotin and Avidin-CY3, samples were incubated with mouse monoclonal anti-Xmr (1:1,000; (Calenda *et al*., 1994)) antibodies that were detected with secondary goat antimouse FITC-conjugated antibodies (Dianova).

Processed samples were evaluated using a Zeiss Axiophot epifluorescence microscope equipped with 63x and 100x plan-neofluoar lenses and with single and double band pass filters for excitation of red, green and blue fluorescence (Chroma Technologies, Battleborough, VT). Digital black-and-white images were recorded with a cooled CCD camera (Hamamatsu), merged to RGB-images by the ISIS fluorescence image analysis system (MetaSystems) and processed in Adobe Photoshop 3.0.

### g) Generation and purification of GST-fusion proteins

The GST-Suvl(82-412) product expressed from the pGEX-2T vector (Pharmacia) as a glutathione-S-transferase (GST) fusion protein has been described (Aagaard *et al*., 1999). Additional GST constructs were generated by transferring BamHI-EcoRI PCR amplicons into pGEX-2T, encoding in-frame fusions for 5UV39H1(82-412), Suv39h2(157-477), CLR4(127-490) (Ivanova *et al*., 1998), EZH2(382-747) (Laible *et al*., 1997) and HRX(3643-3969) (Tkachuk *et al*., 1992). All constructs were confirmed by sequencing.

Recombinant proteins were expressed in 11 cultures of *E.coli* strain BL21 and solubilized in 10 ml RIPA buffer [(20 mM Tris pH 7.5, 500 mM NaCl, 5 mM EDTA, 1% NP-40, 0.5% sodium deoxycholate) containing a full set of protease inhibitors (Boehringer Mannheim) and lysozyme (5 mg/ml; Sigma)] by freeze-thawing in liquid N₂, followed by sonication. Soluble proteins were cleared by centrifugation, purified with 800 µl glutathione Sepharose beads (Pharmacia) and washed twice in RIPA buffer. Protein concentration was determined by Coomassie staining of SDS-PAGE gels. Matrix-bound fusion proteins were used immediately for *in vitro* HMTase assays or stored at 4°C.

### h) In vitro histone methyltransferase (HMTase) assay

*In vitro* HMTase reactions were modified based on described protocols (Strahl *et al*., 1999) and carried out in a volume of 50 µl of methylase activity buffer (MAB: 50 mM Tris pH 8.5, 20 mM KCl, 10 mM MgCl₂, 10 mM β-ME, 250 mM sucrose), containing 10 µg of free histones (mixture of H1, H3, H2B, H2A and H4; Boehringer Mannheim) as substrates and 300 nCi S-adenosyl-[*methyl*-¹⁴C]-L-methionine (25 mCi/ml) (Amersham) as methyl donor. 10 µg of matrix-bound GST-fusion proteins were routinely used to assay for HMTase activity. After incubation for 60 min. at 37°C, reactions were stopped by boiling in SDS loading buffer, and proteins were separated by 15% or 18% SDS-PAGE and visualised by Coomassie staining and fluorography.

### i) Generation of Suv39h1 and Suv39h2 deficient mice by gene targeting

*Suv39h1* maps to the X-chromosome. The cloned partial *Suv39h1* genomic locus was used to generate a targeting construct. A1.2kb Pfu PCR amplicon, generated with the primers gM3-9(SII) and gM3-9(RI), was used as a short arm of homology and cloned in frame with the nls-lacZ gene of the pGNA-T vector. This places the first 3 amino acids of exon 2 in frame with the nls-lacZ gene generating a fusion protein of the first 8 amino acids of Suv39h1 and lacZ from the targeted locus. A 5.4kb SacI (filled in) fragment from *Suv39h1* genomic subclone *gSuv39h1* #18 was used as a long arm of homology.

*Suv39h2 is* autosomal and maps to chromosome 2. The cloned partial *Suv39h2* genomic locus was used to generate a targeting construct. A1.4 kb Pfu PCR amplicon, generated with the primers Suv2SII and Suv2RI, was used as a short arm of homology and cloned in frame with the nls-lacZ gene of the pGNA-T vector. This places the first 113 amino acids of exon 2 in frame with the nls-lacZ gene generating a fusion protein of the first 113 amino acids of Suv39h2 and lacZ from the targeted locus. A 4.9kb MluI/ApaI (filled in) fragment from *Suv39h2* genomic subclone *gSuv39h2* #28 was used as long arm of homology to inactivate the locus.

These constructs were linearised with NotI, electroporated into R1 ES cells (*Suv39h1*) and E14.1 ES cells *(Suv39h2),* ES cells were put under G418 selection and G418 resistant colonies screened for homologous recombination by PCR and Southern blot analysis. Targeted feeder dependent ES cell clones were injected into blastocysts of C57BL/6 mice and reimplanted into pseudopregnant females to produce chimeric offspring. Germ-line transmission was obtained after a backcross between chimeric males and C57BL/6 females. Heterozygous mice were interbred to obtain *Suv39h1* and *Suv39h2* deficient mice. *Suv39h1* and *Suv39h2* deficient mice were then interbred to generate *Suv39h* double deficient mice.

### Example 1

The coding part and conceptional reading frame of the *Suv39h2* cDNA.

To identify additional mammalian *Su(var)3-9* homologues, sequence similarity searches (Bassett *et al*., 1995; Altschul *et al*., 1997) with the murine *Suv39h1* or human *SUV39H1* cDNAs (Aagaard *et al*., 1999) revealed the presence of related, yet distinct expressed sequence tags (ESTs) in DDBJ/EMBL/GenBank databases. In particular, the mouse ESTs fall into two categories that are either homologous to *Suv39h1/SUV39H1* or indicative of a second mammalian *Su(var)3-9* homologue. Using oligonucleotides specific for this second class of *Suv39h*-ESTs, an internal (lacking the conserved chromo and SET domain sequences) DNA probe was PCR-amplified from murine cDNAs and screened against a mouse embryonic day 11.5 cDNA library (see Materials and Methods). Out of six positive isolates, the longest insert was subcloned and sequenced, revealing a nearly full-length open reading frame which comprises the chromo and the C-terminal SET domain. RACE-amplifications with cDNA templates from the murine B-cell specific cell lines J558L and S194 extended the missing 5' end, however, did not detect a starting ATG. To obtain more sequence information, a partial *Suv39h2* genomic clone of approximately 14 kb was isolated (see Materials and Methods). Comparison of the available genomic, cDNA and EST sequences for the *Suv39h1*-related gene allowed the definition of exon 1 (see Materials and Methods) that contains a consensus ATG preceded by in-frame stop codons and which can correctly splice into exon 2. In analogy to *Suv39h1*, this novel gene was designated *Suv39h2* (for *Su(var)3-9* homologue 2). The nucleotide sequence (∼ 1.5 kb) and conceptional reading frame (477 amino acids) of the composite coding *Suv39h2* cDNA is shown in Figure 1.

Fig. 1 shows the ∼ 1.5 kb nucleotide sequence and conceptional reading frame of the coding part of the *Suv39h2* cDNA. Exon1, including the starting ATG preceded by in-frame stop codons (asterisks), has been derived from genomic *Suv39h2* sequences and from an EST that correctly spliced into exon 2. From the available genomic sequences, exons 1-3 could be identified, and their respective exon/intron boundaries are indicated by arrowheads at nucleotide positions 278, 424 and 1083. The 477 amino acids Suv39h2 protein contains several conserved sequence motifs, including a chromo domain (dashed box), the SET domain (grey underlaying) and a C-terminal tail (darker grey bars). Basic amino acids in the N-terminal extension are highlighted by grey circles. In addition, cysteine residues that are also conserved in Suv39h1 are circled. Putative nuclear localisation signals are underlined.

### Example 2

Conserved domains of *S.pombe*, *C.elegans*, *Drosophila* and murine SU(VAR)3-9 related proteins.

Over the length of the 477 amino acids protein, Suv39h2 is 59% identical to Suv39h1 (412 amino acids; (Aagaard *et al*., 1999)). Suv39h2 contains a highly basic (20.7%) N-terminal extension of 82 amino acids that is not present in Suv39h1, although a very basic N-terminus is also found in the C15H11.5 ORF. In addition to its obvious resemblance with protamines, the Suv39h2 N-terminus shows moderate sequence identity (23.2%) with the C-terminal half of the linker histone H1 that is not restricted to basic residues (data not shown). With the exception of this extended N-terminus, Suv39h2 maintains all other conserved domains outlined previously for Suv39h1 (Aagaard *et al*., 1999). For example, both proteins display highest identity in the 130 amino acid SET domain core (75.2%) and at the conspicuous C-terminal tail (69.6%) with its three conserved cysteine residues. Highly identical is also the 60 amino acids chromo domain (62.7%), the SET-associated cysteine-rich region (54.9%) and the 'SU(VAR)3-9 specific' N-terminus (45.0%). In agreement with Suv39h1, Suv39h2 is also significantly shorter as compared to the 635 amino acids fly protein. Alignment of all five representative SU(VAR)3-9 related proteins revealed that among these conserved sequence motifs only the characteristic chromo and SET domains and the C-terminal tail are shared by all family members. By contrast, the SET-associated cysteines are absent in the *C. elegans* C15H11.5 ORF and less than half of the SET-adjacent, cysteine-rich region appears conserved. The highest variation is observed at the N-termini, with SU(VAR)3-9 containing a 155 amino acid extension including a putative GTP binding site (Tschiersch *et al*., 1994), CLR4 lacking any sequences preceding the chromo domain, and with Suv39h2 and the C15H11.5 ORF encoding very basic, yet distinct N-terminal extensions.

Fig. 2 illustrates the phylogenetic relationships of murine Suv39h1 (412 amino acids), murine Suv39h2 (477 amino acids), *Drosophila* SU(VAR)3-9 (635 amino acids), *S.pombe* CLR4 (490 amino acids) and a *C.elegans* ORF C15H11.5 (503 amino acids). Over the entire length of the protein, Suv39h1 shares 59% identity with Suv39h2, 41% identity with SU(VAR)3-9, 35% identity with CLR4 and 18% identity with C15H11.5. Similarly, Suv39h2 shares 59% identity with Suv39h1, 39% identity with SU(VAR)3-9, 37% identity with CLR4 and 22% identity with C15H11.5. Highly conserved sequence motifs are indicated, and comprise the chromo (box filled with vertical lines) and SET (black) domains, and the SET-associated cysteine-rich clusters (grey) which are only in part present in C15H11.5. In addition, an N-terminal region (box filled with horizontal lines) shared by the murine and fly proteins (Aagaard *et al*., 1999), a putative GTP-binding domain (dot filled box) (Tschiersch *et al*., 1994) in SU(VAR)3-9 and the basic N-termini (box filled with diagonal lines) in Suv39h2 and C15H11.5 are also highlighted.

### Example 3

Expression of *Suv39h1* and *Suv39h2* during mouse development.

Abundant *Suv39h2*-specific transcripts are present in ES-cells, in *in vitro* differentiated embryoid bodies (EB) and between day E10.5 - day E15.5, with embryonic expression peaking around day E10.5. In contrast, *Suv39h2* transcripts are substantially down-regulated at day E17.5 and are nearly absent during postnatal development. A very similar dynamic expression profile was also observed for *Suv39h1*, with the exception that the relative abundance of *Suv39h1* transcripts in ES-cells and embryoid bodies is reduced as compared to *Suv39h2* transcripts (Figure 3, top panel). To investigate the spatial expression profiles of *Suv39h2* and *Suv39h1,* whole-mount *in-situ* hybridisations with *Suv39h2-* and *Suv39h1*-specific riboprobes (see Materials and Methods) was performed on day E8.5 and day E9.5 mouse embryos. Whereas only residual staining is observed with a *Suv39h2* control sense probe, the *Suv39h2* antisense probe reveals a rather uniform expression throughout the entire embryos (data not shown). Similarly, the *Suv39h1* antisense probe detects a broad distribution of transcripts, consistent with the ubiquitous expression of *Suv39h1* in previous *in-situ* hybridisations on sagittal sections of day E12.5 embryos (Aagaard *et al*., 1999). In addition to embryonic tissues, the mesenchyme-derived allantois is also prominently stained by the *Suv39h1* antisense probe (data not shown). Together with the RNA blot shown above, this comparative analysis indicates significant co-expression and potential overlapping functions during mouse development for *Suv39h1* and *Suv39h2.*

Fig. 3 shows the₋RNA blot analysis to detect *Suv39h1* and *Suv39h2* transcripts in 15µg of total RNA prepared from undifferentiated CCE embryonic stem cells (ES), embryoid bodies (EB) derived after retinoic acid-induced *in vitro* differentiation of CCE cells, and whole embryos at various stages of embryonic (E10.5 - E17.5) and postnatal development (P1- P4). As a control for the quality of the RNA, the RNA blot was re-hybridised with a probe that is specific for *Gapdh* sequences.

### Example 4

Testis-specific expression of *Suv39h2*.

The abundance of *Suv39h2* and *Suv39h1* transcripts greatly differs in adult tissues. Whereas *Suv39h1* displays broad expression in a panel of RNA preparations comprising 14 adult tissues, expression of *Suv39h2* remains largely restricted to testes, with mRNAs being present as 2.7 kb and 1.7 kb transcripts. In addition to other tissues, *Suv39h2* transcripts are also significantly down-regulated in ovaries. To analyse this testis-specific expression in more detail, *in-situ* hybridisations on sections of adult testes were performed. The *Suv39h2* and *Suv39h1* antisense probes revealed specific expression in the outermost cell layer of the seminiferous tubules (data not shown), whereas the corresponding control sense probes proved negative. *Suv39h2*-specific transcripts appear at elevated levels as compared to *Suv39h1*. Higher magnification (data not shown) shows predominant staining of type B spermatogonia and pre-leptotene spermatocytes. *Suv39h2*-specific transcripts are also detected at reduced levels in several pachytene-stage cells and in mitotically inactive Sertoli cells (data not shown). Together, these data indicate a prominent expression of *Suv39h2* transcripts in male germ cells during the early stages of spermatogenesis and are suggestive of a function for *Suv39h2* in male gametogenesis.

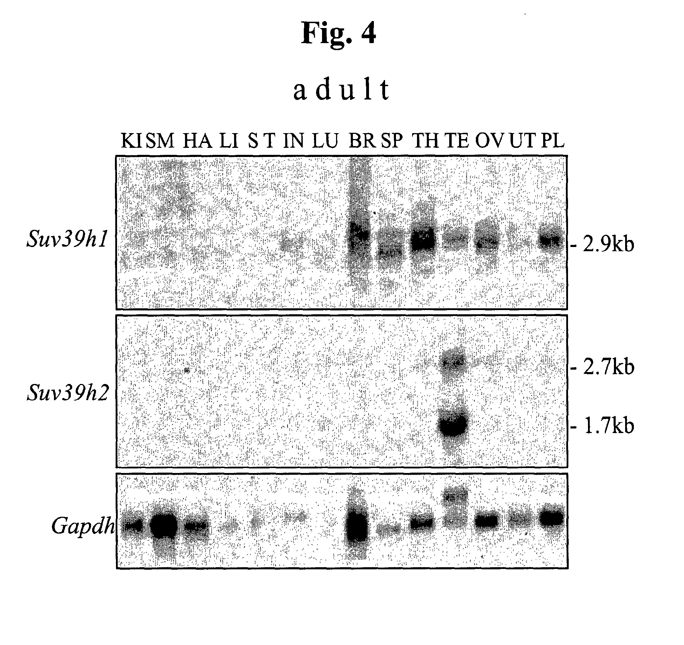

Fig. 4 shows the₋RNA blot analysis to detect *Suv39h1* and *Suv39h2* transcripts in 15µg of total RNA prepared from adult 129/Sv tissues, including kidney (KI), skeletal muscle (SM), heart (HA), liver (LI), stomach (ST), intestine (IN), lung (LU), brain (BR), spleen (SP), thymus (TH), testis (TE), ovaries (OV), uterus (UT) and placenta (PL). As a loading control, the RNA blot was re-hybridised with a probe that is specific for *Gapdh* sequences.

### Example 5

Generation of anti-sera specific for Suv39h2.

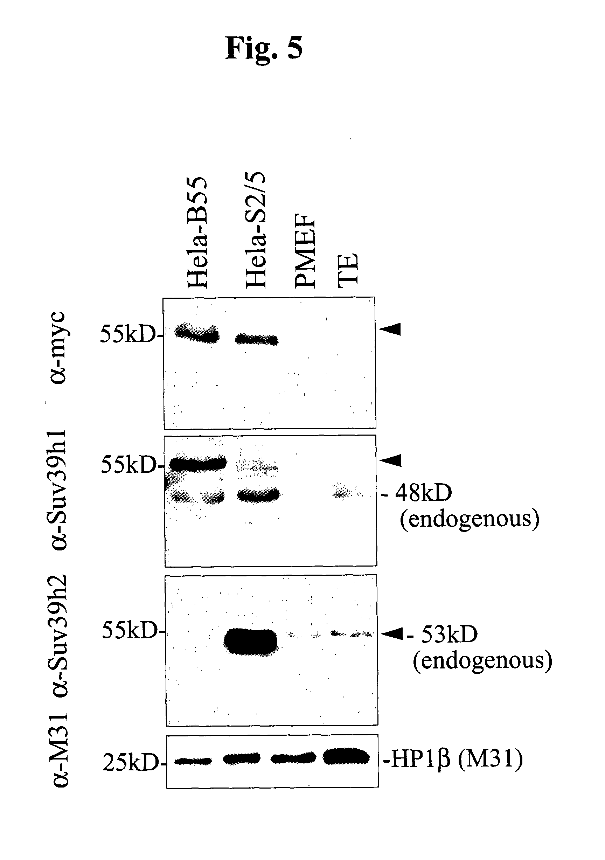

To characterise Suv39h2 expression at a biochemical level, a polyclonal rabbit antiserum was generated that was raised against a recombinant glutathione *S*-transferase (GST) fusion protein comprising amino acids 157-477 of murine Suv39h2. This serum was preabsorbed against the related GST-Suv39h1 antigen (Aagaard *et al*., 1999) and affinity-purified (see Materials and Methods). Western blot analysis of *in-vitro* translated Suv39h2 and human SUV39H1 (which is 95% identical to murine Suv39h1; (Aagaard *et al*., 1999)) indicated that the anti-Suv39h2 antiserum specifically recognised the Suv39h2 gene product but largely failed to detect the endogenous protein in a variety of mammalian cell lines (data not shown). Therefore, protein blots containing nuclear extracts from primary mouse embryonic fibroblasts (PMEFs) and from adult testis were probed with anti-Suv39h1 and anti-Suv39h2 antibodies. As a specificity and size control, nuclear extracts from HeLa cell lines that 'stably' overexpress (myc)₃-SW39H1 (HeLa-B55; 40) or a corresponding (myc)3-Suv39h2 construct which encodes amino acids 83-477 of the Suv39h2 cDNA (HeLa-S2/5) (see Materials and Methods) were included. Immunoblotting with anti-Suv39h1 antibodies indicated the presence of ectopic (myc)3-SUV39H1 (55 kDa) and of endogenous SUV39H1 (48 kDa) in HeLa-B55 nuclear extracts. However, endogenous Suv39h1 was undetectable in PMEFs and only low-abundant in testis (Figure 5, middle panel). By contrast, the anti-Suv39h2 antibodies recognise an endogenous protein of approximately 53 kDa in both PMEFs and testis (Figure 5, lower panel), which co-migrates with ectopic (myc)3-Suv39h2(83-477) in HeLa-S2/5 nuclear extracts. It was concluded that Suv39h2 is more highly expressed in PMEFs and testis than Suv39h1, and that the size of the endogenous Suv39h2 protein is in good agreement with the gene product predicted from the coding sequence of the *Suv39h2* cDNA (see Figure 1).

In the experiment shown in Fig. 5, approximately 30 µg of nuclear extracts from HeLa-B55, HeLa-S2/5, primary mouse fibroblasts (PMEFs) and adult testis (TE) were immunoblotted with anti-myc, anti-Suv39h1, anti-Suv39h2 and anti-M31 (as a loading control) antibodies. HeLa-B55 overexpress (myc)3-SUV39H1(3-412) and HeLa-S2/5 overexpress (myc)₃-Suv39h2(83-477). The size of these ectopic proteins is indicated by arrowheads. Endogenous Suv39h2 (53 kDa) co-migrates with (myc)3-Suv39h2(83-477). The anti-Suv39h1 and anti-Suv39h2 antibodies are specific for their respective epitopes and do not cross-react.

### Example 6

Dynamic heterochromatin association of Suv39h2 during most stages of spermatogenesis.

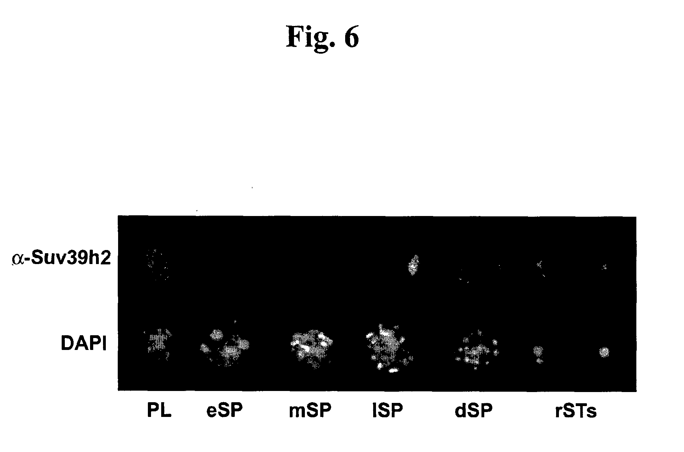

The subnuclear localisation endogenous Suv39h2 protein in nuclei of testis swab preparations was analysed (see Materials and Methods) by indirect immunofluorescence with the anti-Suv39h2 antibodies. Possible chromosomal associations in structurally preserved suspension cells that comprised early to late stages of spermatogenesis were examined. Endogenous Suv39h2 is found in a dispersed distribution in some pre-meiotic nuclei (data not shown) and as a granular stain in all pre-leptotene nuclei (Figure 6, left nucleus). During the development of leptotene to diplotene spermatocytes, Suv39h2 staining is weakly but distinctly apparent at blocks of heterochromatin, as visualised by the bright DAPI counterstaining. Surprisingly, prominent Suv39h2 signals accumulate at the sex chromosomes present in the XY body during mid-pachytene (see below). After the meiotic divisions, Suv39h2 remains enriched at the condensing heterochromatic foci of haploid spermatids (Figure 6, right nuclei), but is no longer detectable in mature sperm (data not shown).

Fig. 6 shows the indirect immunofluorescence of testis suspension cells with anti-Suv39h2 antibodies. DNA was counterstained with DAPI (bottom panel). Staging of individual mouse spermatogenic cells was determined as described in Materials and Methods and comprised pre-leptotene spermatogonia (PL), early, middle and late spermatocytes (eSP, mSP, 1SP), diplotene spermatocytes (dSP), and round spermatids (rST).

### Example 7

Suv39h2 accumulates with sex chromosomes present in the X-Y body.

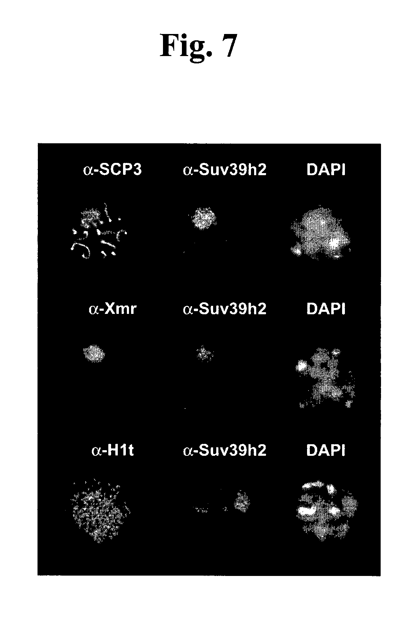

To demonstrate the specific accumulation of Suv39h2 with the sex chromosomes, double immunofluorescence analyses for Suv39h2 and SCP3, and for Suv39h2 and Xmr was performed. SCP3 stains the axial cores of the synaptonemal complex (SC) which is formed during homologue pairing of autosomes (Lammers *et al*., 1994). By contrast, the Xmr protein selectively associates with the axes and chromatin of sex chromosomes (Calenda *et al*., 1994), which are enclosed in the XY body during pachytene and whose pairing is delayed relative to the autosomes. The results of these co-localisations show that the concentration of the Suv39h2 signal overlaps with a diffuse SCP3 staining around the unpaired axes of the sex chromosomes but not with the SC of paired autosomes (Figure 7, top panel). Moreover, Suv39h2 co-localises with the XY body, as defined by Xmr staining and the presence of unpaired axial cores of the sex chromosomes (Figure 7, middle panel). These data indicate that Suv39h2 specifically accumulates with chromatin of the sex chromosomes in late prophase of meiosis I. To more definitively determine the timing and differentiation stage at which Suv39h2 accumulates with the sex chromosomes, our analysis was extended by double immunofluorescence for Suv39h2 and the testis-specific histone H1 variant H1t (Meistrich, 1987). H1t appears in mid-pachynema and is detected until haploid spermatids reach the elongation stage. In developing spermatocytes, H1t therefore defines spermatocytes I from mid-pachytene to diplotene (Moens, 1995). Analysis of Hlt-stained pachytene nuclei revealed the simultaneous presence of a Suv39h2-positive XY body (Figure 7, bottom panel), indicating specific association of Suv39h2 with sex chromosomes from mid-late pachytene to diplotene.

Fig. 7:shows the results of double-labelling indirect immunofluorescence for Suv39h2 and either SCP3 (top panel), Xmr (middle panel), or histone H1t (bottom panel) in mid-pachytene to diplotene spermatocytes of adult testis suspensions. DNA was counterstained with DAPI.

### Example 8

Suv39h2 harbours HMTase activity.

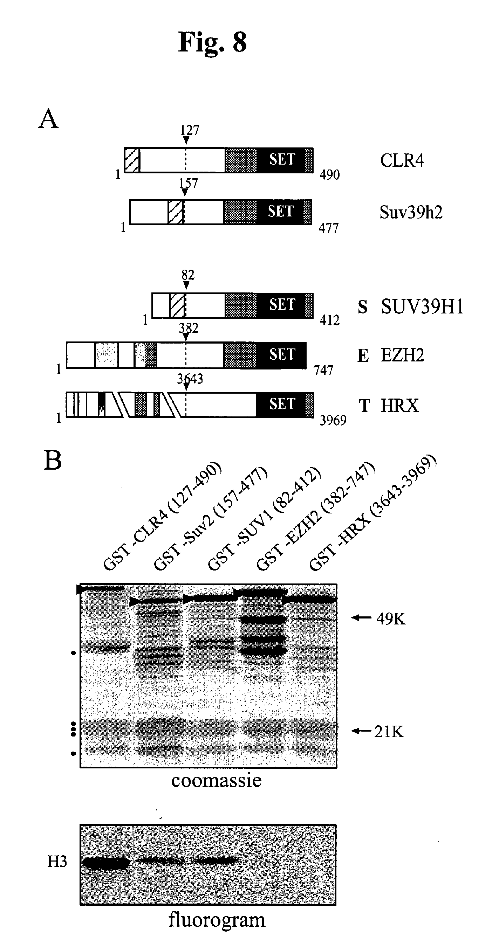

The SET domains of SU(VAR)3-9 protein family shares significant sequence and secondary structure with six plant MTases (Rea *et al*., 2000). Because the SET domain is one of the most conserved protein motifs in chromatin regulators (Stassen *et al*., *1995;* Jenuwein *et al*., 1998), it was analyzed whether SU(VAR)3-9 family members or other SET domain proteins contain HMTase activity. GST-fusion products of the extended SET domains of murine Suv39h2, *S.pombe* CLR4 (Ivanova *et al*., 1998), human EZH2 (Laible *et al*., 1997) and human HRX (Tkachuk *et al*., 1992) were generated that would correspond to GST-SL7V39H1(82-412) and assayed for HMTase activity. The SU(VAR)3-9 family members assayed, SUV39H1, Suv39h2 and CLR4, displayed HMTase activity. By contrast, both GST-EZH2(382-747) and GST-HRX(3643-3966) had undetectable HMTase activity towards free histones (Figure 8b).

Fig. 8A shows a diagram representing the domain structures of CLR4, Suv39h2, SUV39H1, EZH2 and HRX proteins, with the arrowheads demarcating the N-terminal fusion to GST. Cysteine-rich regions are indicated by grey stippling.

In the experiment of Fig. 8B, approximately 10 µg of the indicated fusion proteins encoding *S.pombe* CLR4 [GST-CLR4(127-490)], murine Suv39h2 [GST-Suv2(157-477)], human EZH2 [GST-EZH2(382-747)], human HRX [GST-HRX(3643-3969)] and human SUV39H1 [GST-SUV1(82-402)] were used in *in vitro* HMTase reactions with free histones as outlined in the materials and methods.

### Example 9

Targeting the *Suv39h1* and *Suv39h2* loci in the mouse germline.

Murine *Suv39h* genes are encoded by 2 loci, *Suv39h1* and *Suv39h2*.To investigate the *in vivo* significance of *Suv39h* function and *Suv39h* dependent K9 H3 methylation, mouse strains deficient for both *Suv39h1* and *Suv39h2* were generated according to standard techniques. The targeting strategies are shown in Figure 9, as well as demonstrating the production of null alleles for both *Suv39h1* and *Suv39h2.* Mutation of either gene results in viable and fertile mice as a consequence of functional redundancy between both loci. Therefore, *Suv39h1* and *Suv39h2* deficient strains were intercrossed to produce *Suv39h* double deficient mice. Double mutant mice are born in sub-Mendelian ratios, approximately 20% of the expected double mutants are observed.

Fig. 9 shows the conventional targeting strategy used to inactivate the X-linked *Suv39h1* locus. Fig. 9B shows the Northern blot analysis of *Suv39h1* from spleen (Sp), liver (Li), kidney (Kidney), and brain (Br) from wild-type and *Suv39h1* null mice. Fig. 9 shows the conventional targeting strategy used to inactivate the autosomal *Suv39h2* locus. (Bottom panel) Western blot analysis with anti-Suv39h2 antibodies on protein extracts derived from wild-type and *Suv39h2* null testis.

### Example 10

*Suv39h* function is required for male gametogenesis.

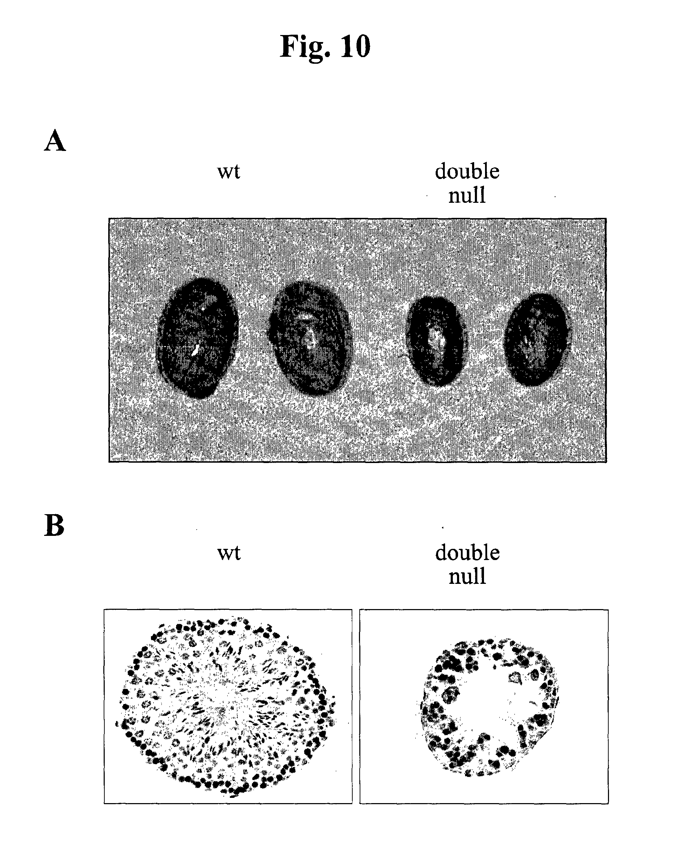

In the experiments conducted, it was observed that surviving double mutants are growth retarded and display hypogonadism (Figure 10a) accompanied by apoptotic spermatogonia (data not shown). In the few surviving spermatids, the progressive clustering of centromeres that occurs during spermiogenesis is severely impaired (data not shown). Histological analysis of double mutant testis reveals highly aberrant tubules devoid of mature sperm rendering *Suv39h* double deficient mice infertile (Figure 10b).

Fig 10A: shows testis isolated from a wild-type and a *Suv39h* double null mice. Fig. 10B shows the histological analysis of testis isolated from a wild-type and a *Suv39h* double null mouse. Shown are sections of seminiferous tubules, *Suv39h* double null tubules are devoid of mature sperm.

### References

Aagaard, L., Laible, G., Selenko, P., Schmid, M., Dorn, R., Schotta, G., Kuhfittig, S., Wolf, A., Lebersorger, A., Singh, P.B., Reuter, G. and Jenuwein, T. (1999) Functional mammalian homologues of the Drosophila PEV-modifier Su(var)3- 9 encode centromere-associated proteins which complex with the heterochromatin component M31. *Embo J,* **18,** 1923-38.
Aagaard, L., Schmid, M., Warburton, P. and Jenuwein, T. (2000) Mitotic phosphorylation of SUV39H1, a novel component of active centromeres, coincides with transient accumulation at mammalian centromeres. *J Cell Sci*, **113**, 817-829.
Aasland, R. and Stewart, A.F. (1995) The chromo shadow domain, a second chromo domain in heterochromatin- binding protein 1, HP1. *Nucleic Acids Res,* **23,** 3168-74.
Allshire, R.C., Nimmo, E.R., Ekwall, K., Javerzat, J.P. and Cranston, G. (1995) Mutations derepressing silent centromeric domains in fission yeast disrupt chromosome segregation. *Genes Dev*, **9,** 218-33.
Altschul, S.F., Madden, T.L., Schaffer, A.A., Zhang, J., Zhang, Z., Miller, W. and Lipman, D.J. (1997) Gapped BLAST and PSI-BLAST: a new generation of protein database search programs. *Nucleic Acids Res*, **25,** 3389-402.
Ball, L.J., Murzina, N.V., Broadhurst, R.W., Raine, A.R.C., Archer, S.J., Stott, F.J., Murzin, A.G., Singh, P.B., Domaille, P.J. and Laue, E.D. (1997) Structure of the chromatin binding (chromo) domain from mouse modifier protein 1. *EMBO J*, **16**, 2473-2481.
Bassett, D.E., Jr., Boguski, M.S., Spencer, F., Reeves, R., Goebl, M. and Hieter, P. (1995) Comparative genomics, genome cross-referencing and XREFdb. *Trends Genet,* **11,** 372-3.
Boulianne, G. L., et al., (1984), Nature 312: 643-646
Bunick, D., Johnson, P.A., Johnson, T.R. and Hecht, N.B. (1990) Transcription of the testis-specific mouse protamine 2 gene in a homologous in vitro transcription system. *Proc Natl Acad Sci USA*, **87,** 891-5.
Calenda, A., Allenet, B., Escalier, D., Bach, J.F. and Garchon, H.J. (1994) The meiosis-specific Xmr gene product is homologous to the lymphocyte Xlr protein and is a component of the XY body. *Embo J,* **13,** 100-9.
Dernburg, A.F., Sedat, J.W. and Hawley, R.S. (1996) Direct evidence for a role of heterochromatin in meiotic chromosome segregation. *Cell,* **86**, 135-146.
Dugaiczyk, A., Haron, J.A., Stone, E.M., Dennison, O.E., Rothblum, K.N. and Schwartz, R.J. (1983) Cloning and sequencing of a deoxyribonucleic acid copy of glyceraldehyde-3-phosphate dehydrogenase messenger ribonucleic acid isolated from chicken muscle. *Biochemistry,* **22,** 1605-13.
Gentz *et al*., *Proc. Natl. Acad. Sci. USA 86*:821-824 (1989), hexa-histidine provides for convenient purification of the fusion protein.
Graziano, R.F., et al., (1995), J. Immunol. 155: 4996-5002
Handel, M.A. and Hunt, P.A. (1992) Sex-chromosome pairing and activity during mammalian meiosis. *Bioessays*, **14,** 817-22.
Henikoff, S. (1997) Position effect variegation in Drosophila: recent progress. *Epigenetic mechanisms of gene regulation.* CSHL press.
Ivanova, A.V., Bonaduce, M.J., Ivanov, S.V. and Klar, A.J. (1998) The chromo and SET domains of the Clr4 protein are essential for silencing in fission yeast. *Nat Genet,* **19**, 192-5.
Jenuwein, T., Laible, G., Dorn, R. and Reuter, G. (1998) SET domain proteins modulate chromatin domains in eu- and heterochromatin. *Cell Mol Life Sci,* **54**, 80-93.
Karpen, G.H. and Allshire, R.C. (1997) The case for epigenetic effects on centromere identity and function. *TIG*, **13**, 489-496.
Köhler, G. und Milstein, C. (1975), Nature 265, 495-497
Koonin, E.V., Zhou, S. and Lucchesi, J.C. (1995) The chromo superfamily: new members, duplication of the chromo domain and possible role in delivering transcription regulators to chromatin. *Nucleic Acids Res,* **23,** 4229-33.
Kot, M.C. and Handel, M.A. (1990) Spermatogenesis in XO,Sxr mice: role of the Y chromosome. *J Exp Zool,* **256,** 92-105.
Laible, G., Wolf, A., Dorn, R., Reuter, G., Nislow, C., Lebersorger, A., Popkin, D., Pillus, L. and Jenuwein, T. (1997) Mammalian homologues of the Polycomb-group gene Enhancer of zeste mediate gene silencing in Drosophila heterochromatin and at S. cerevisiae telomeres. *Embo J,* **16,** 3219-32.
Lammers, J.H., Offenberg, H.H., van Aalderen, M., Vink, A.C., Dietrich, A.J. and Heyting, C. (1994) The gene encoding a major component of the lateral elements of synaptonemal complexes of the rat is related to X-linked lymphocyte-regulated genes. *Mol Cell Biol*, **14**, 1137-46.
Matsuda, Y., Hirobe, T. and Chapman, V.M. (1991) Genetic basis of X-Y chromosome dissociation and male sterility in interspecific hybrids. *Proc Natl Acad Sci U S A*, **88**, 4850-4.
Meistrich, M.L.a.B., W. A. (1987) Proteins of the meiotic cell nucleus. In Moens, P.B. (ed.) *Meiosis.* Academic Press, New York, N.Y.
Melcher, M., Schmid, M., Aagaard, L., Selenko, P., Laible, G. and Jenuwein, T. (2000) Structure-function analysis of SUV39H1 reveals a dominant role in heterochromatin organization, chromosome segregation, and mitotic progression. *Mol Cell Biol*, **20,** 3728-41.
Messmer, S., Franke, A. and Paro, R. (1992) Analysis of the functional role of the Polycomb chromo domain in Drosophila melanogaster. *Genes Dev,* **6,** 1241-54.
Moens, P.B. (1995) Histones H1 and H4 of surface-spread meiotic chromosomes. *Chromosoma*, **104,** 169-74.
Motzkus, D., Singh, P.B. and Hoyer-Fender, S. (1999) M31, a murine homolog of Drosophila HP1, is concentrated in the XY body during spermatogenesis. *Cytogenet Cell Genet,* **86,** 83-8.
Neuberger, M.S., et al., (1984), Nature 312: 604-608
Pandita, T.K., Westphal, C.H., Anger, M., Sawant, S.G., Geard, C.R., Pandita, R.K. and Scherthan, H. (1999) Atm inactivation results in aberrant telomere clustering during meiotic prophase. *Mol Cell Biol,* **19,** 5096-105.
Paro, R. and Harte, P.J. (1996) *The role of Polycomb group and thrithorax group complexes in the maintenance of determined cell states.* Coldspring Harbour Laboratory Press, New York, NY.
Paro, R. and Hogness, D.S. (1991) The Polycomb protein shares a homologous domain with a heterochromatin- associated protein of Drosophila. *Proc Natl Acad Sci USA*, **88**, 263-7.
Platero, J.S., Hartnett, T. and Eissenberg, J.C. (1995) Functional analysis of the chromo domain of HP1. *Embo J,* **14,** 3977-86.
Rea, S., Eisenhaber, F., O'Carroll, D., Strahl, B.D., Sun, Z., Schmid, M., Opravil, S., Mechtler, K., Ponting, C.P., Allis, C.D. and Jenuwein, T. (2000) Regulation of chromatin structure by site-specific histone H3 methyltransferases. *In press Nature.*
Reuter, G. and Spierer, P. (1992) Position effect variegation and chromatin proteins. *BioEssays*, **14,** 605-612.
Riechmann, L., et al., (1988), Nature 332: 323-327
Sambrook, J., Fritsch, E.F. and Maniatis, T. (1989) *Molecular cloning: A laboratory manual.* Cold Spring Harbour Laboratory Press, New York.
Scherthan, H., Weich, S., Schwegler, H., Heyting, C., Harle, M. and Cremer, T. (1996) Centromere and telomere movements during early meiotic prophase of mouse and man are associated with the onset of chromosome pairing. *J Cell Biol,* **134**, 1109-25.
Solari, A.J. (1974) The behavior of the XY pair in mammals. *Int Rev Cytol*, **38,** 273-317.
Stassen, M.J., Bailey, D., Nelson, S., Chinwalla, V. and Harte, P.J. (1995) The Drosophila trithorax proteins contain a novel variant of the nuclear receptor type DNA binding domain and an ancient conserved motif found in other chromosomal proteins. *Mech Dev*, **52,** 209-23.
Strahl, B.D., Ohba, R., Cook, R.G. and Allis, C.D. (1999) Methylation of histone H3 at lysine 4 is highly conserved and correlates with transcriptionally active nuclei in Tetrahymena. *Proc Natl Acad Sci U S A*, **96,** 14967-72.
Tkachuk, D.C., Kohler, S. and Cleary, M.L. (1992) Involvement of a homolog of Drosophila trithorax by 11q23 chromosomal translocations in acute leukemias. *Cell,* **71,** 691-700.
Tschiersch, B., Hofmann, A., Krauss, V., Dorn, R., Korge, G. and Reuter, G. (1994) The protein encoded by the Drosophila position-effect variegation suppressor gene Su(var)3-9 combines domains of antagonistic regulators of homeotic gene complexes. *Embo J,* **13,** 3822-31.
Wallrath, L.L. (1998) Unfolding the mysteries of heterochromatin. *Curr Opin Genet Dev*, **8**, 147-53.
Wilson *et al*., *Cell 37*: 767 (1984).
Wreggett, K.A., Hill, F., James, P.S., Hutchings, A., Butcher, G.W. and Singh, P.B. (1994) A mammalian homologue of Drosophila heterochromatin protein 1 (HP1) is a component of constitutive heterochromatin. *Cytogenet Cell Genet,* **66**, 99-103.

## Claims

1. Murine Suv39h2 polypeptide with the amino acid sequence as set forth in SEQ ID NO:2 or with the amino acid sequence encoded by a polynucleotide which hybridises under stringent conditions to a polynucleotide having a nucleotide sequence as set forth in SEQ ID NO: 1.

2. An isolated DNA molecule comprising a polynucleotide with the nucleotide sequence as set forth in SEQ ID NO: 1 encoding murine Suv39h2 polypeptide or an isolated DNA molecule encoding murine Suv39h2, comprising a polynucleotide which hybridises under stringent conditions to a polynucleotide having a nucleotide sequence as set forth in SEQ ID NO: 1.

3. Human SUV39H2 polypeptide encoded by a polynucleotide containing the sequence of the human EST accession number AQ173625 (SEQ ID NO:3) and/or AQ494637 (SEQ ID NO:4) and/or AQ691972 (SEQ ID NO:5) and/or AQ554070 (SEQ ID NO:6), or by a polynucleotide which hybridises under stringent conditions to the said polynucleotides.

4. An isolated DNA molecule encoding the human SUV39H2 polypeptide, comprising a polynucleotide containing the sequence of the human EST accession number AQ173625 (SEQ ID NO:3) and/or AQ494637 (SEQ ID NO:4) and/or AQ691972 (SEQ ID NO:5) and/or AQ554070 (SEQ ID NO:6), or an isolated DNA molecule.

5. An antibody against murine Suv39h2.

6. An antibody against human SUV39H2.

7. A method for identifying a compound that has the ability of modulating mammalian male gametogenesis, wherein one or more Suv39h/SUV39H homologues required for male gametogenesis are incubated, in the presence of the substrate(s) for the histone methyltransferase activity of the Suv39h/SUV39H homologue and in the presence of a methyl donor, with a test compound and that the modulating effect of the test compound on the histone methyltransferase activity of the Suv39h/ SUV39H homologue(s) is determined.

8. The method of claim 7, wherein the Suv39h/ SUV39H homologue is Suv39h2/SUV39H2.

9. The method of claim 8, wherein in a first step, the Suv39h/SUV39H homologue is Suv39h2/SUV39H2, and a compound identified as an inhibitor or activitor of Suv39h2/SUV39H2 is, in a second step, confirmed to be also an inhibitor or activator of Suv39h1/SUV39H1 histone methyltransferase activity.

10. The method of any one of claims 7 to 9, wherein the substrate is histone H3 or the N-terminal fragment thereof that contains the methylation site at lysine 9.

11. The method of claim 10, wherein the histone H3 N-terminal fragment has the amino acid sequence as set forth in SEQ ID NO:7.

12. The method of any one of claims 7 to 11, wherein the methyl donor is radioactively labelled methionine or S-adenosyl-L-methionine.

13. The method of claim 12, wherein the methyl donor carries a chromogenic label and the methyltransferase activity is determined by measuring the change in colour upon transfer of the methyl group to the subtrate.

14. The method of claim 12, wherein the methyl donor carries a radioactive label and the methyltransferase activity is determined by measuring the radioactivity transferred to the substrate upon transfer of the methyl group.

15. The method of any one of claims 7 to 11, wherein the methyltransferase activity of the Suv39h/SUV39H homologue is determined immunologically by quantifying the binding of an antibody specific for the methylation site to the substrate.

16. A compound identified in a method of any one of claims 7 to 15 to be an inhibitor of the Suv39h/SUV39H histone methyltransferase activity for male contraception.


SEQUENCE LISTING

<110> Boehringer Ingelheim International GmbH

<120> Novel Suv3-9 homologue

<130> Case 14/056

<140>
<141>

<160> 11

<170> PatentIn Ver. 2.1

<210> 1
<211> 1452
<212> DNA
<213> Mus musculus

<220>
<221> 3'UTR
<222> (1)..(18)

<220>
<221> CDS
<222> (19)..(1452)

<400> 1

gaatgaaagc tccgcaag atg gcg acg gcc agg gcc aag gca cgg ggc agt 51
Met Ala Thr Ala Arg Ala Lys Ala Arg Gly Ser
1               5                   10

gag gca gga gcg cgg tgt cac cgg gct cca ggt ccg ccc ccg agg ccc 99
Glu Ala Gly Ala Arg Cys His Arg Ala Pro Gly Pro Pro Pro Arg Pro
            15                  20                  25

aag gcc agg cga acg gcg aga cgc cgc cgc gcg gag acc ctg acg gcg 147
Lys Ala Arg Arg Thr Ala Arg Arg Arg Arg Ala Glu Thr Leu Thr Ala
        30                  35                  40

cga cgc tcg cgg ccg tct gcg ggc gag agg cgc gcc ggc tcc cag cga 195
Arg Arg Ser Arg Pro Ser Ala Gly Glu Arg Arg Ala Gly Ser Gln Arg
    45                  50                  55

gcg tgg tcc gga gct ccg cgg gcc gcg gtc ttt ggc gac gag tgt gca 243
Ala Trp Ser Gly Ala Pro Arg Ala Ala Val Phe Gly Asp Glu Cys Ala
60                  65                  70                  75

cga ggt gcc tta ttc aag gcc tgg tgt gtg cct tgc cta gtt tca ctt 291
Arg Gly Ala Leu Phe Lys Ala Trp Cys Val Pro Cys Leu Val Ser Leu
                80                  85                  90

gat act ctc cag gaa tta tgt aga aaa gaa aag ctc aca tgt aaa tcg 339
Asp Thr Leu Gln Glu Leu Cys Arg Lys Glu Lys Leu Thr Cys Lys Ser
            95                  100                 105

att gga atc acc aaa agg aat cta aac aat tat gag gtg gag tac ttg 387
Ile Gly Ile Thr Lys Arg Asn Leu Asn Asn Tyr Glu Val Glu Tyr Leu
        110                 115                 120

tgt gac tac aag gta gca aag ggt gtg gaa tat tat ctt gta aaa tgg 435
Cys Asp Tyr Lys Val Ala Lys Gly Val Glu Tyr Tyr Leu Val Lys Trp
    125                 130                 135

aaa gga tgg cca gat tct aca aac acc tgg gag ccc ttg aga aac ctc 483
Lys Gly Trp Pro Asp Ser Thr Asn Thr Trp Glu Pro Leu Arg Asn Leu
140                 145                 150                 155

agg tgt cca cag ctc ctg cgg cag ttc tct gat gac aag aag act tac 531
Arg Cys Pro Gln Leu Leu Arg Gln Phe Ser Asp Asp Lys Lys Thr Tyr
                160                 165                 170

tta gct cag gaa agg aaa tgc aag gct gtc aat tca aaa tcc ttg caa 579
Leu Ala Gln Glu Arg Lys Cys Lys Ala Val Asn Ser Lys Ser Leu Gln
            175                 180                 185

cct gca att gct gag tat att gta cag aaa gct aag caa aga ata gct 627
Pro Ala Ile Ala Glu Tyr Ile Val Gln Lys Ala Lys Gln Arg Ile Ala
        190                 195                 200

ctg cag aga tgg caa gat tac ctc aac aga aga aag aac cat aag ggg 675
Leu Gln Arg Trp Gln Asp Tyr Leu Asn Arg Arg Lys Asn His Lys Gly
    205                 210                 215

atg ata ttt gtt gaa aac act gtt gac ttg gag ggc cca cct tta gac 723
Met Ile Phe Val Glu Asn Thr Val Asp Leu Glu Gly Pro Pro Leu Asp
220                 225                 230                 235

ttc tac tac att aac gag tac agg cca gct ccc ggg atc agc ata aac 771
Phe Tyr Tyr Ile Asn Glu Tyr Arg Pro Ala Pro Gly Ile Ser Ile Asn
                240                 245                 250

agt gaa gcc acc ttt gga tgt tca tgt aca gac tgc ttc ttt gac aag 819
Ser Glu Ala Thr Phe Gly Cys Ser Cys Thr Asp Cys Phe Phe Asp Lys
            255                 260                 265

tgt tgt cct gct gaa gct gga gtt gtg ttg gct tat aat aag aag caa 867
Cys Cys Pro Ala Glu Ala Gly Val Val Leu Ala Tyr Asn Lys Lys Gln
        270                 275                 280

caa att aaa atc caa cca ggc act ccc atc tac gaa tgc aac tca agg 915
Gln Ile Lys Ile Gln Pro Gly Thr Pro Ile Tyr Glu Cys Asn Ser Arg
    285                 290                 295

tgt cga tgt gga cct gaa tgt ccc aat agg att gta caa aaa ggc aca 963
Cys Arg Cys Gly Pro Glu Cys Pro Asn Arg Ile Val Gln Lys Gly Thr
300                 305                 310                 315

caa tat tca ctg tgc atc ttt aaa act agc aat ggc tgt ggt tgg ggt     1011
Gln Tyr Ser Leu Cys Ile Phe Lys Thr Ser Asn Gly Cys Gly Trp Gly
                320                 325                 330

gta aaa acc ctt gtg aag att aaa aga atg agt ttt gtc atg gaa tat     1059
Val Lys Thr Leu Val Lys Ile Lys Arg Met Ser Phe Val Met Glu Tyr
            335                 340                 345

gtt gga gag gtg atc aca agt gaa gag gcc gag aga cgg gga cag ttc     1107
Val Gly Glu Val Ile Thr Ser Glu Glu Ala Glu Arg Arg Gly Gln Phe
        350                 355                 360

tat gac aac aaa ggg atc acc tac ctc ttt gac ctg gac tac gag tct     1155
Tyr Asp Asn Lys Gly Ile Thr Tyr Leu Phe Asp Leu Asp Tyr Glu Ser
    365                 370                 375

gat gag ttc aca gtg gat gca gct cga tat gga aac gta tcc cat ttt     1203
Asp Glu Phe Thr Val Asp Ala Ala Arg Tyr Gly Asn Val Ser His Phe
380                 385                 390                 395

gtg aat cat agt tgt gac cca aat ctt cag gtg ttt agt gtt ttc atc     1251
Val Asn His Ser Cys Asp Pro Asn Leu Gln Val Phe Ser Val Phe Ile
                400                 405                 410

gat aac ctt gat act cgg ctg ccc agg ata gca ttg ttc tct aca aga     1299
Asp Asn Leu Asp Thr Arg Leu Pro Arg Ile Ala Leu Phe Ser Thr Arg
            415                 420                 425

acc ata aac gct gga gaa gag ctg act ttt gac tat caa atg aaa ggt     1347
Thr Ile Asn Ala Gly Glu Glu Leu Thr Phe Asp Tyr Gln Met Lys Gly
        430                 435                 440

tct gga gaa gca tct tca gac tcc att gac cac agc cct gcc aaa aaa     1395
Ser Gly Glu Ala Ser Ser Asp Ser Ile Asp His Ser Pro Ala Lys Lys
    445                 450                 455

agg gtc aga acc caa tgt aaa tgt gga gcc gag act tgc aga ggt tac     1443
Arg Val Arg Thr Gln Cys Lys Cys Gly Ala Glu Thr Cys Arg Gly Tyr
460                 465                 470                 475

ctc aac tga                                                         1452
Leu Asn

<210> 2
<211> 477
<212> PRT
<213> Mus musculus

<400> 2

Met Ala Thr Ala Arg Ala Lys Ala Arg Gly Ser Glu Ala Gly Ala Arg
  1               5                  10                  15

Cys His Arg Ala Pro Gly Pro Pro Pro Arg Pro Lys Ala Arg Arg Thr
             20                  25                  30

Ala Arg Arg Arg Arg Ala Glu Thr Leu Thr Ala Arg Arg Ser Arg Pro
        35                  40                  45

Ser Ala Gly Glu Arg Arg Ala Gly Ser Gln Arg Ala Trp Ser Gly Ala
    50                  55                  60

Pro Arg Ala Ala Val Phe Gly Asp Glu Cys Ala Arg Gly Ala Leu Phe
65                  70                  75                  80

Lys Ala Trp Cys Val Pro Cys Leu Val Ser Leu Asp Thr Leu Gln Glu
                85                  90                  95

Leu Cys Arg Lys Glu Lys Leu Thr Cys Lys Ser Ile Gly Ile Thr Lys
            100                 105                 110

Arg Asn Leu Asn Asn Tyr Glu Val Glu Tyr Leu Cys Asp Tyr Lys Val
        115                 120                 125

Ala Lys Gly Val Glu Tyr Tyr Leu Val Lys Trp Lys Gly Trp Pro Asp
    130                 135                 140

Ser Thr Asn Thr Trp Glu Pro Leu Arg Asn Leu Arg Cys Pro Gln Leu
145                 150                 155                 160

Leu Arg Gln Phe Ser Asp Asp Lys Lys Thr Tyr Leu Ala Gln Glu Arg
                165                 170                 175

Lys Cys Lys Ala Val Asn Ser Lys Ser Leu Gln Pro Ala Ile Ala Glu
            180                 185                 190

Tyr Ile Val Gln Lys Ala Lys Gln Arg Ile Ala Leu Gln Arg Trp Gln
        195                 200                 205

Asp Tyr Leu Asn Arg Arg Lys Asn His Lys Gly Met Ile Phe Val Glu
    210                 215                 220

Asn Thr Val Asp Leu Glu Gly Pro Pro Leu Asp Phe Tyr Tyr Ile Asn
225                 230                 235                 240

Glu Tyr Arg Pro Ala Pro Gly Ile Ser Ile Asn Ser Glu Ala Thr Phe
                245                 250                 255

Gly Cys Ser Cys Thr Asp Cys Phe Phe Asp Lys Cys Cys Pro Ala Glu
            260                 265                 270

Ala Gly Val Val Leu Ala Tyr Asn Lys Lys Gln Gln Ile Lys Ile Gln
        275                 280                 285

Pro Gly Thr Pro Ile Tyr Glu Cys Asn Ser Arg Cys Arg Cys Gly Pro
    290                 295                 300

Glu Cys Pro Asn Arg Ile Val Gln Lys Gly Thr Gln Tyr Ser Leu Cys
305                 310                 315                 320

Ile Phe Lys Thr Ser Asn Gly Cys Gly Trp Gly Val Lys Thr Leu Val
                325                 330                 335

Lys Ile Lys Arg Met Ser Phe Val Met Glu Tyr Val Gly Glu Val Ile
            340                 345                 350

Thr Ser Glu Glu Ala Glu Arg Arg Gly Gln Phe Tyr Asp Asn Lys Gly
        355                 360                 365

Ile Thr Tyr Leu Phe Asp Leu Asp Tyr Glu Ser Asp Glu Phe Thr Val
    370                 375                 380

Asp Ala Ala Arg Tyr Gly Asn Val Ser His Phe Val Asn His Ser Cys
385                 390                 395                 400

Asp Pro Asn Leu Gln Val Phe Ser Val Phe Ile Asp Asn Leu Asp Thr
                405                 410                 415

Arg Leu Pro Arg Ile Ala Leu Phe Ser Thr Arg Thr Ile Asn Ala Gly
            420                 425                 430

Glu Glu Leu Thr Phe Asp Tyr Gln Met Lys Gly Ser Gly Glu Ala Ser
        435                 440                 445

Ser Asp Ser Ile Asp His Ser Pro Ala Lys Lys Arg Val Arg Thr Gln
    450                 455                 460

Cys Lys Cys Gly Ala Glu Thr Cys Arg Gly Tyr Leu Asn
465                 470                 475

<210> 3
<211> 543
<212> DNA
<213> Homo sapiens

<220>
<221> misc_feature
<222> (1)..(543)
<223> EST Acc. No.173625

<400> 3

ggccatgtgg ttgancccct ggntttaccn nnccntggnn ggnnttgann ccccttagat 60
tatagtccag aatcattgtt gtcatataac tgccctcatc tttcagcttc gtcacttgtg 120
attacctttc caacttattc catgacaaaa cttattcttt taatcttcac atgggttttt 180
acaccccagc catggtcatt gatactgtga aagatgcaaa gtgaattact gtgtgccttt 240
ttgtacaatc ctattggtac agtgaggtcc acattgacag attgagatgc atttatagat 300
gggagtaaca ggtgggattt taatttgttg gtttttacta taagccaaaa gaattccagc 360
ttcaccaaga caacattttt catagaagca atctgtgcat gaacaacaaa aggtagcttc 420
atttactaag ctgattccag gagctggttt gtattcatca atatagcaga agtctgaagg 480
tgggccttct aagtgaaccc tattntcaac aaatatcact cctttattat tctgtcttct 540
gcg                                                               543

<210> 4
<211> 579
<212> DNA
<213> Homo sapiens

<220>
<221> misc_feature
<222> (1)..(579)
<223> EST Acc. No. AQ494637

<400> 4

gcttctcata catgatacgt gttcngctct gnngtntnng tttangaata cntaaaanaa 60
aaggnagggg ngncntttga ttcgtgtgat tccatagatg cactcatatg gaactgtatt 120
tcattntgtg aatcatagta gtgacccaaa tcttcatatg ttctatgntn tcactgataa 180
cttgacactg gccttcccta tatagctctg tgttccatga gaactataaa tgctggagaa 240
gagttgattt ttgacaatca aacaaaaagt tctggggata tatcttcaga gtttattgac 300
cacagctcag ccaaaaagag ggtcagaact gtatgtaaat gtggagctgt gacttgcaga 360
ggttgcctca aatgaatttt caggaaatag aaatgatgat aattggtagt tgtttctttt 420
ttctaatgtt atcattctaa aaataagtat ttggaactct cttttcatat tatcaagatt 480
attactatgt taaattgaca tncatggttc aaggcattta ccanatgcat tactgatgcc 540
tcttgagaga gggccactgt gttgcataga ctgatctga 579

<210> 5
<211> 565
<212> DNA
<213> Homo sapiens

<220>
<221> misc_feature
<222> (1)..(565)
<223> EST Acc. No. AQ691972

<400> 5

agaggatgag catggatcnt cgctatagca aaccacanat anaatcccac ctgttactcc 60
catctataaa tgcatctcaa tctgtcaatg tggaccttac tgtaccaata ggattgtaca 120
aaaaggcaca cagtaattca ctttgcatct ttcacagtat caatgaccat ggctggggtg 180
taaaaaccca tgtgaagatt aaaagaataa gttttgtcat ggaataagtt ggaaaggtaa 240
tcacaagtga cgaagctgaa agatgagggc agttatatga caacaaatga tctggactat 300
gaatctgatg aattcacaga ggatgcagct caatatggaa ctgtatttca ttntgtgaat 360
cataagtagt gacccaaact tcatatgttc aatgttntca ttgataactt gacactggcc 420
tttccttaat agctctgtgt tccatgagaa ctataaatgc tggagaagaa gtgatttttg 480
acatcaacaa aagttctggg attatcttca aagttattgc cacagttacc aaaagaaggc 540
aaactgttgt aatgtgagct gtact 565

<210> 6
<211> 535
<212> DNA
<213> Homo sapiens

<220>
<221> misc_feature
<222> (1)..(535)
<223> EST Acc. No. AQ554070

<400> 6

tcagactcat agtccagatc aaagagattc tgtgattccc ttgttgtcat agaactgtcc 60
tcgtctttca gcttcttcac ttgtgattac ctaaacagaa aaaactgtaa gtatattacg 120
tagctactga accaaagaag cattcatcta cctatctact aatatgcgaa tacctacaaa 180
tatttaaaaa gtaagaaatt caggtgtcat caaagcaaac attcacacaa actaagactc 240
agatgcaaag aggtgggaaa atgaggggaa gaaaaatgat aatgcaaaag actgatgacc 300
tttttttttt aaacagggtc tcactctgtc actcaggcta gaatgcggtg gtgccatcat 360
gactccctgt atcctttaac tcctgggatc aagcgatctt cctgcctcag cctcctgact 420
agctggatca caggtgcata ccgccatgcc cagctaatga tttagttttt atagagatgt 480
ggggtctcac tatgttgccc acactggtct ggaactcctg ggctcaagtg agcct 535

<210> 7
<211> 20
<212> PRT
<213> Homo sapiens

<400> 7

Ala Arg Thr Lys Gln Thr Ala Arg Lys Ser Thr Gly Gly Lys Ala Pro
1               5                   10                  15

Arg Lys Gln Leu
            20

<210> 8
<211> 24
<212> DNA
<213> Artificial sequence

<220>
<223> Primer

<400> 8

GGGGATGATA TTTGTTGAAA ACAC 24

<210> 9
<211> 24
<212> DNA
<213> Artificial sequence

<220>
<223> Primer

<400> 9

GGTTGGATTT TAATTTGTTG CTTC 24

<210> 10
<211> 19
<212> DNA
<213> Artificial sequence

<220>
<223> Primer

<400> 10

GCCCTCCAAG TCAACAGTG 19

<210> 11
<211> 22
<212> DNA
<213> Artificial sequence

<220>
<223> Primer

<400> 11

GTGTTGAGGT AATCTTGCCA TC 22